# EUROPEAN PATENT APPLICATION

(11) **EP 2 295 591 A1**
(43) Date of publication of application: **16.03.2011**
(21) Application number: 09745870.7
(22) Date of filing: 13.05.2009
(51) Int. Cl.: C12N 15/861, C12N 15/85

(54) **SELF-INACTIVATING HELPER ADENOVIRUSES FOR THE PRODUCTION OF HIGH-CAPACITY RECOMBINANT ADENOVIRUSES**

(30) Priority: 16.05.2008 ES 200801434
(71) Applicant: Proyecto de Biomedicina Cima, S.L., 31008 Pamplona - Navarra (ES)
(72) Inventor: GONZÁLEZ APARICIO, Manuela, E-31008 Pamplona - Navarra (ES); HERNÁNDEZ ALCOCEBA, Rubén, E-31008 Pamplona - Navarra (ES); MAULEON MAYORA, Miren Itsaso, E-31008 Pamplona - Navarra (ES)
(74) Representative: ABG Patentes, S.L.
(86) International application number: PCT/ES2009/070154
(87) International publication number: WO 2009/138544

(57) **Abstract**

The invention relates to reagents and methods for the generation of auto-inactivating helper adenoviruses that allow the development of cell systems for the generation of high-capacity recombinant adenovirus showing a reduced contamination by auxiliary adenoviruses.

SEQUENCE LISTING

<110>  Centro de Investigación Médica Aplicada (CIMA)

<120>  SELF-INACTIVATING HELPER ADENOVIRUSES FOR THE PRODUCTION OF
       HIGH-CAPACITY RECOMBINANT ADENOVIRUSES

<130>  P3809PC00

<150>  P200801434.7
<151>  2008-05-16

<160>  6

<170>  PatentIn version 3.5

<210>  1
<211>  24
<212>  DNA
<213>  Artificial sequence

<220>
<223>  primer 5Ad5St

<400>  1
tagtgtggcg gaagtgtgat gttg
24


<210>  2
<211>  37
<212>  DNA
<213>  Artificial sequence

<220>
<223>  primer AdpTGC-10

<400>  2
tgactagtat aagcggccgc agcagatacg ggatatc
37


<210>  3
<211>  37
<212>  DNA
<213>  Artificial sequence

<220>
<223>  primer 5SwaB

<400>  3
ctgactgagc ggccgcattt aaatgtcgac acttcac
37


<210>  4
<211>  37
<212>  DNA

<213>  Artificial sequence

<220>
<223>  primer 3SwaB

<400>  4
gtgaagtgtc gacatttaaa tgcggccgct cagtcag
37


<210>  5
<211>  38
<212>  DNA
<213>  Artificial sequence

<220>
<223>  primer 5ClaITR

<400>  5
actgacatcg atttaattaa catcatcaat aatatacc
38


<210>  6
<211>  31
<212>  DNA
<213>  Artificial sequence

<220>
<223>  primer 3ClaLoxP

<400>  6
tagattatcg attcttaacc acgcccagat c
31

## Description

### Field of the Invention

The invention relates to the field of adenoviral vectors and, more specifically, to the field of production systems of third-generation or gutless adenoviral vectors by means of the use of self-inactivating helper adenoviruses which allow the generation of recombinant adenoviruses in cells with a minimum contamination of the high-capacity adenovirus by helper adenoviruses.

### Background of the Invention

High-capacity adenoviruses (HC-Ad), also known as gutless adenovirus or helper-dependent adenovirus, have a great appeal as vectors for gene therapy because they maintain the high transduction efficiency and tropism of other adenoviral vectors both in rodents and in primates, but they prevent the immunogenicity associated to the expression of viral proteins in infected cells. In addition, these viral vectors have a great capacity for inserting a transgene of interest (up to approximately 36 Kb), much higher than that of other viral vectors.

High-capacity adenoviral vectors are based on adenoviruses into which all the viral coding sequences were "emptied". These vectors have as minimum requirements the terminal regions of the adenoviral genome (ITR, inverted terminal repeats) which are necessary for the viral replication, and the packaging signal (ψ), which also acts in *cis.*

Given that high-capacity adenoviruses lack all the viral coding regions, the presence of a helper virus deleted in the E1 region, which provides viral proteins in *trans,* is required for their propagation. Propagation is carried out by means of co-transfection or co-infection in 293 cell lines, in which the deleted E1 region in the helper virus has been integrated.

However, with this propagation system unwanted viral particles which encapsidate helper virus genomes and contaminate the final preparations of the high-capacity adenoviruses are also generated. Helper virus accumulation is quite variable, but on occasions it can be so intense that it displaces the high-capacity virus, this being one of the reasons for which current production methods are not reliable enough for clinical use. The performance is another limiting factor, also partly related to the problem of the appearance of helper viruses. Until now different systems for reducing the packaging efficiency of the helper virus have been developed and described. These systems are generally based on the incorporation of mutations in the packaging signal of the helper virus, on the increased size of its genome, and more particularly on a specific elimination of the packaging signal during the viral production process.

Parks et al. (Proc. Natl. Acad. Sci. USA 1996; 93:13565-13570) have described methods for the elimination of the packaging signal ψ of the helper virus by means of a Cre-loxP recombination system, in which the signal ψ of said virus is flanked by loxP sites. If propagation is carried out in 293 cells expressing Cre recombinase, the recombinase excises the signal ψ of the helper virus, preventing it from being able to be packaged, but such that it retains the coding sequences necessary for the packaging of the high-capacity adenovirus. Alternatively, US 7,045,347 and Ng et al. (Mol Ther 2001; 3: 809-815) have described inactivation systems of the helper virus based on helper viruses the packaging sequence of which is flanked by frt recognition sites for FLP recombinase, such that the propagation of the helper virus in 293 cells expressing yeast FLP recombinase results in the excision of the packaging sequence.

These systems have the drawback that the reaction catalyzed by the recombinase is bidirectional, whereby the packaging sequence can be reintroduced again in the genome of the helper virus. To prevent this problem, Groth et al. (Proc. Natl. Acad. Sci. USA 2000; 97:5995-6000) have proposed the use of a unidirectional recombinase, particularly PhiC31 recombinase, using specific attB/attP recognition sequences to flank the signal ψ. When PhiC31 recombinase excises the sequence flanked by attB/attP sites, it modifies the recognition sequences and converts them into attR/attL sequences, preventing the recognition from occurring again (with the reintroduction of the signal ψ).

However, all the methods are faced with the problem that the elimination of the packaging sequences of the helper virus requires the expression of a recombinase from its coding sequence located in the genome of the host cell. Said process occurs in a moment of the viral cycle in which the expression of cellular proteins is partially inhibited due to the cytopathic effects associated to infection by adenoviruses, therefore it is possible that high enough intracellular recombinase levels to eliminate all the copies of helper adenovirus are not reached.

Therefore, alternative methods have been proposed to prevent the generation of helper adenoviruses with capacity to be packaged or, at least, to delay the formation thereof with regard to the formation of gutless vectors. Patent document WO200712514 thus describes a variant of the previously described method in which helper adenoviral vectors comprise a phage ΦC31 attB sequence between the packaging sequence and the ITR closest to said packaging sequence. The inclusion of said attB site results, even in the absence of ΦC31 recombinase, in a delay in the packaging of the helper adenovirus with regard to the gutless adenovirus, which causes a reduction of the amount of contaminating helper adenoviruses in the preparation of gutless viruses. However, this method only results in a delay in the formation of the helper adenoviruses but it does not prevent the formation of helper adenoviruses with packaging capacity.

This problem has been solved by means of the adenoviral system described in DE10159167. In this system, the recombinase is encoded by the gutless adenovirus and the helper adenovirus contains recombinase recognition target sites which delimit a region comprising the packaging signal and the elimination of which results in E1 adenoviral gene, present in the helper adenovirus in 3' direction with regard to an expression silencer, being close to an AAV p5 transcription promoter. Therefore, in the presence of the gutless adenovirus, the recombinase eliminates the packaging region of the genome of the helper adenovirus while at the same time allowing expression of the E1 gene, which is necessary for the packaging of the gutless adenovirus. However, this adenoviral system includes a helper virus expressing E1, therefore it is a replicative virus and, therefore, more dangerous than a conventional helper virus. Furthermore, the gutless virus expresses recombinase, which is unwanted in a therapeutic vector with potential clinical use.

Another alternative solution for preventing the dependence of the recombinase from the packaging cell is the use of a recombinase encoded by the helper virus itself. Patent document US20020072120 describes helper adenoviral vectors encoding a fusion protein formed by Cre recombinase and the ligand binding domain of the estrogen receptor, said fusion protein being encoded by a sequence which is under the operative control of a constitutive promoter. The Cre-ER fusion protein only translocates to the nucleus and exerts its recombinase activity in the presence of an estrogen or analog, whereby eliminating the need for using a cell expressing the recombinase, while at the same time preventing the toxic effect of the recombinase given that its activity can be regulated by means of the addition of estrogens to the culture medium.

Therefore, there is a need in the art for packaging systems for adenoviral vectors which eliminate the drawbacks of the systems known up until now, particularly methods in which the generation of helper adenoviruses during the propagation of the gutless adenovirus is maximally reduced.

### Summary of the Invention

In a first aspect, the invention relates to a polynucleotide comprising
a) a region encoding a site-specific recombinase, wherein said region is under operative control of an inducible promoter and
b) an essential region for the replication and/or the packaging of the adenoviral genome flanked by recognition sequences specific for the recombinase encoded by sequence a), wherein said recognition sequences are oriented such that the excision of said essential region occurs in the presence of said recombinase.

In a second aspect, the invention relates to an expression vector comprising a polynucleotide of the invention.

In a third aspect, the invention relates to an adenoviral particle comprising the sequence of a polynucleotide of the invention

In another aspect, the invention relates to a system for the replication and packaging of a self-inactivating helper adenovirus, comprising:
a) a polynucleotide, an expression vector or an adenoviral particle according to the invention; and
b) a eukaryotic cell.

In another aspect, the invention relates to a method for obtaining a self-inactivating helper adenovirus comprising
(i) contacting a cell with a polynucleotide, an expression vector or an adenoviral particle of the invention and, optionally, with one or several polynucleotides encoding the adenoviral proteins necessary for the packaging and replication of the adenovirus in the cell in suitable conditions so that the entry in the cell of the adenoviral genome or of the polynucleotide comprising said genome occurs,
(ii) keeping the cell in suitable conditions to allow the packaging and replication of the adenovirus in the cell and to prevent the expression of the site-specific recombinase and
(iii) recovering the adenoviruses from the medium.

In another aspect, the invention relates to a helper adenovirus obtained according to the method of the preceding paragraph.

In another aspect, the invention relates to the use of a polynucleotide, of a vector or of an adenoviral particle according to the invention for the production and packaging of high-capacity adenoviruses.

In another aspect, the invention relates to a system for the production of a high-capacity recombinant adenovirus comprising
(a) a polynucleotide, an expression vector or an adenoviral particle according to the invention;
(b) an adenovirus the genome of which comprises a gene of interest and which lacks the sequence encoding at least one of the essential proteins for the replication or the sequence encoding at least one of the essential proteins for the packaging of said adenovirus or with a polynucleotide comprising the genome of said adenovirus and
(c) a eukaryotic cell.

In another aspect, the invention relates to a method for generating a high-capacity adenovirus expressing a gene of interest comprising the steps of
(i) contacting a cell with an adenovirus in suitable conditions so that the entry in the cell of the genome of said adenovirus or of the polynucleotide comprising said genome occurs,
(ii) contacting said cell with a second component selected from the group of:
   (a) a polynucleotide comprising:
      1. a region encoding a site-specific recombinase, wherein said region is under operative control of an inducible promoter and
      2. an adenoviral packaging signal ψ flanked by recognition sequences specific for the recombinase encoded by sequence a), wherein said recognition sequences are oriented such that the excision of the packaging signal ψ occurs in the presence of said recombinase,
   (b) an adenoviral vector comprising a polynucleotide according to (a) and
   (c) an adenoviral particle comprising a polynucleotide according to (a)
      in suitable conditions so that the entry in the cell of said polynucleotide, of said adenoviral vector or of the genome of said adenoviral particle occurs,
(iii) keeping the cell in suitable conditions for the expression of the proteins encoded by the genomes of both viruses, for the replication of the genomes of both adenoviruses and for the expression of the recombinase encoded by the polynucleotide, vector or adenovirus used in step (ii) and
(iv) recovering the high-capacity adenovirus from the cell culture.

### Brief Description of the Drawings

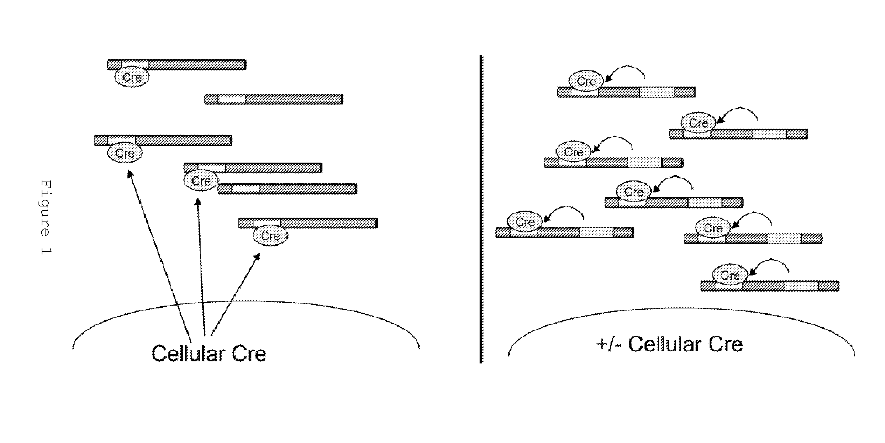

Figure 1 shows an operating scheme of the self-inactivatable helper adenovirus. Panel A shows the classic method based on the production of Cre by the packaging cell. In intense viral replication conditions, the expression of Cre can be insufficient for excising the packaging sequence of a number of viral genomes which is multiplied exponentially. Panel B shows how each self-inactivatable helper virus is a carrier of a Cre gene, such that the expression capacity of Cre increases in parallel to the increase of viral genomes.

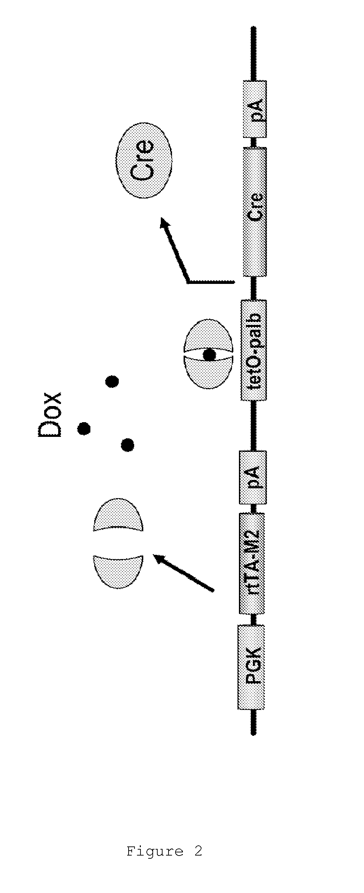

Figure 2 shows an operating scheme of the inducible system for the expression of Cre. A constitutive promoter (PGK) directs the expression of transactivating protein rtTA-M2. In the presence of the inducer (doxycycline), rtTA-M2 dimerizes and activates an inducible promoter (tetO-pAlb) controlling the expression of Cre recombinase. pA, polyadenylation sequence.

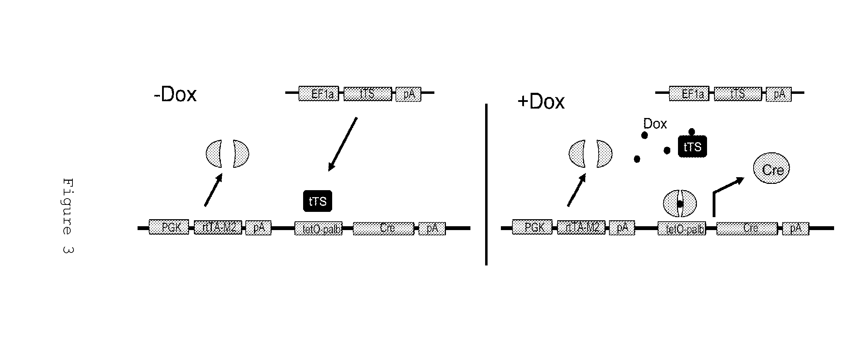

Figure 3 shows the operating scheme of the protein tTS. In the absence of doxycycline, tTS binds to the inducible promoter and inhibits the expression of Cre. When doxycycline is added, tTS loses affinity for the promoter, stops inhibiting the expression of Cre, and allows the dimer-mediated activation of the transactivating protein.

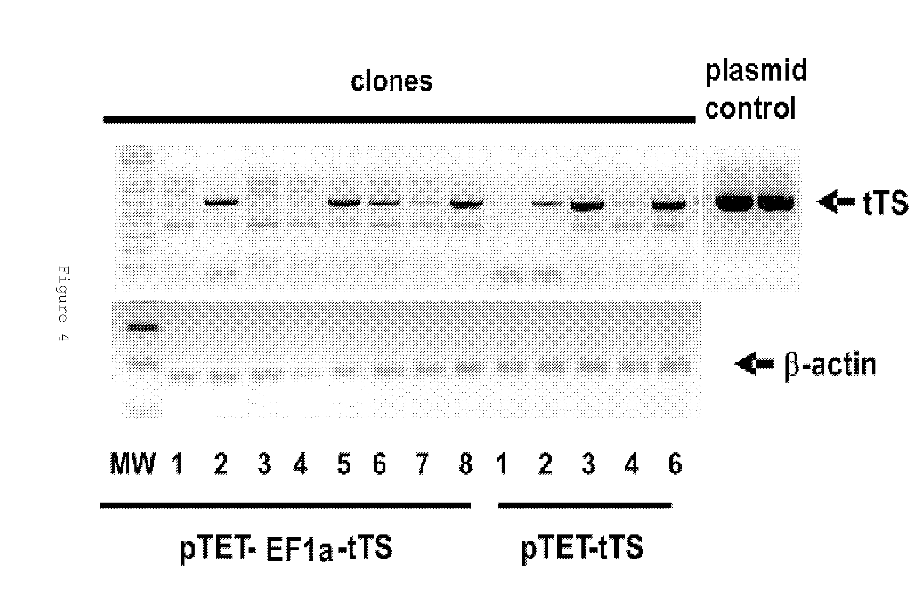

Figure 4 shows the stable expression of tTS in 293 cells. RNA of the different clones of 293 cells transfected with the plasmid expressing tTS was purified, and RT-PCR was carried out to detect the production of tTS mRNA. The plasmids containing cDNA were included as a control. MW, size marker.

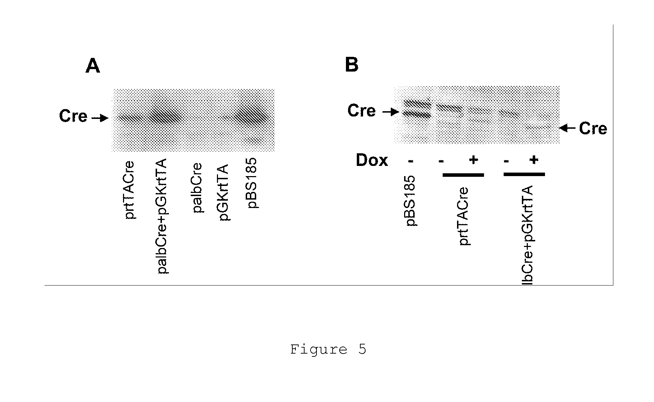

Figure 5 shows the control of the expression of Cre in inducible plasmids. 293 cells were transfected with the indicated plasmids: pBS185 expresses Cre under the control of a constitutive CMV promoter; palbCre contains the inducible promoter by controlling the expression of Cre; pGKrtTA expresses the transactivator under the control of the PGK promoter; prtTACre contains the expression cassettes of Cre and of the transactivator in one and the same plasmid. In A, all the cells were maintained in the presence of doxycycline. In B, they were compared in the presence or absence of doxycycline. The expression of Cre was detected by means of Western Blot.

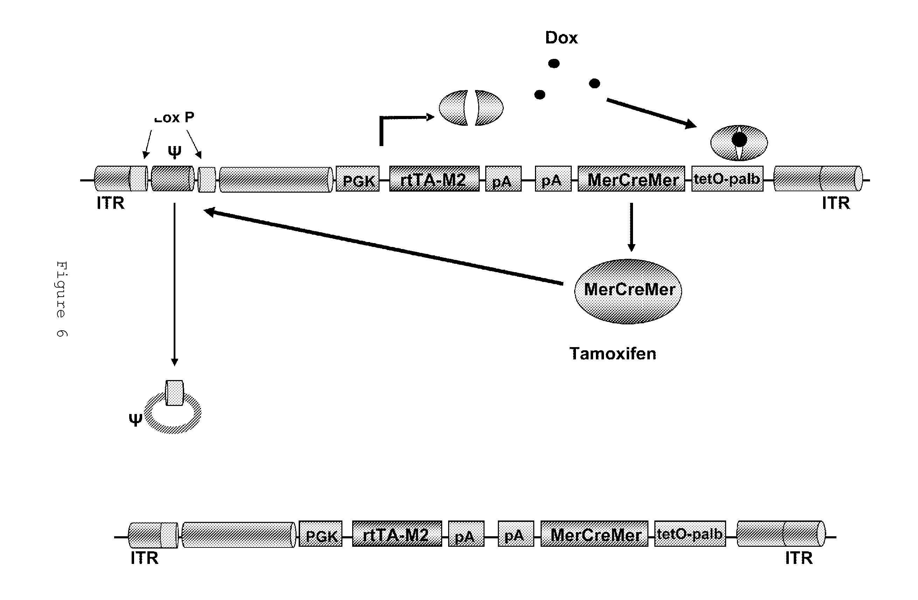

Figure 6 shows a diagrammatic representation of the AdTetCre virus and its functioning. The packaging signal (Ψ) is flanked by LoxP sites in the same orientation. The rtTA-M2 transactivator is under the control of the constitutive promoter of the murine PGK gene and the MerCreMer protein is under the control of its TetO-palb inducible promoter. In the presence of doxycycline the rtTA-M2 transactivator dimerizes and binds to the TetO-palb promoter. This activates the expression of the MerCreMer protein, which will be active in the presence of tamoxifen, recognizing LoxP sites and eliminating the packaging signal to prevent the helper virus from encapsidating. ITR, Inverted Terminal Repeat.

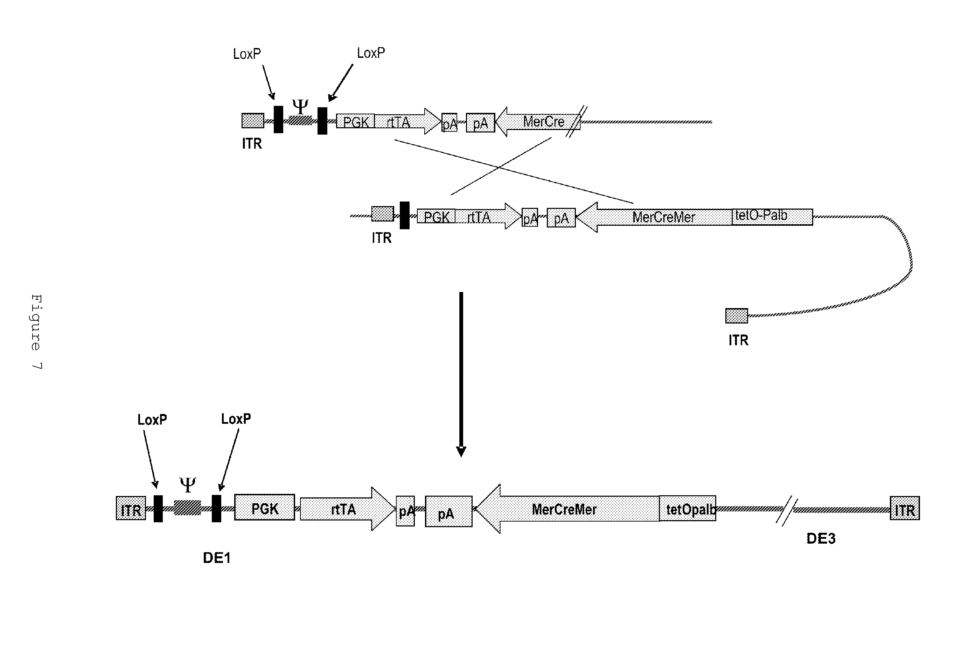

Figure 7 shows the recombination between the pAdLoxPMerCre and pShutMer plasmids to generate the genome of the AdTetCre virus. In 293tTs cells, the pShutMer and pAdLoxPMerCre plasmids both digested with the Pac I restriction enzyme, were co-transfected. The recombination takes place between the homologous areas of both plasmids, the genome of an infective adenovirus being restored with the complete expression cassette for MerCreMer and the packaging signal flanked by LoxP sites.

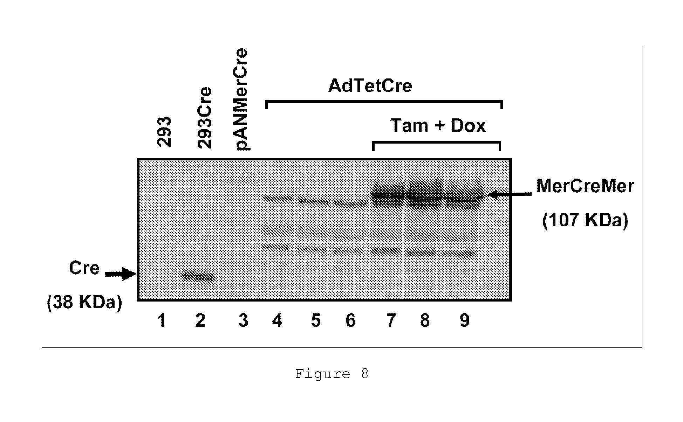

Figure 8 shows the control of the expression of the MerCreMer protein in the AdTetCre virus. 293tTs cells were infected with the AdTetCre virus at an MOI of 2 viruses/cell for 48 hours, in the presence (lines 7-9) or absence (lines 4-6) of treatment with tamoxifen and doxycycline. An extract of 293 cells was loaded in line 2 to verify the detection of the wild-type recombinase with the antibody. 293tTs cells were transfected in line 3 with the pANMerCreMer plasmid. Line 1 corresponds to non-infected 293 cells as a negative control. The expression of Cre and MerCreMer was detected by means of immunoblotting.

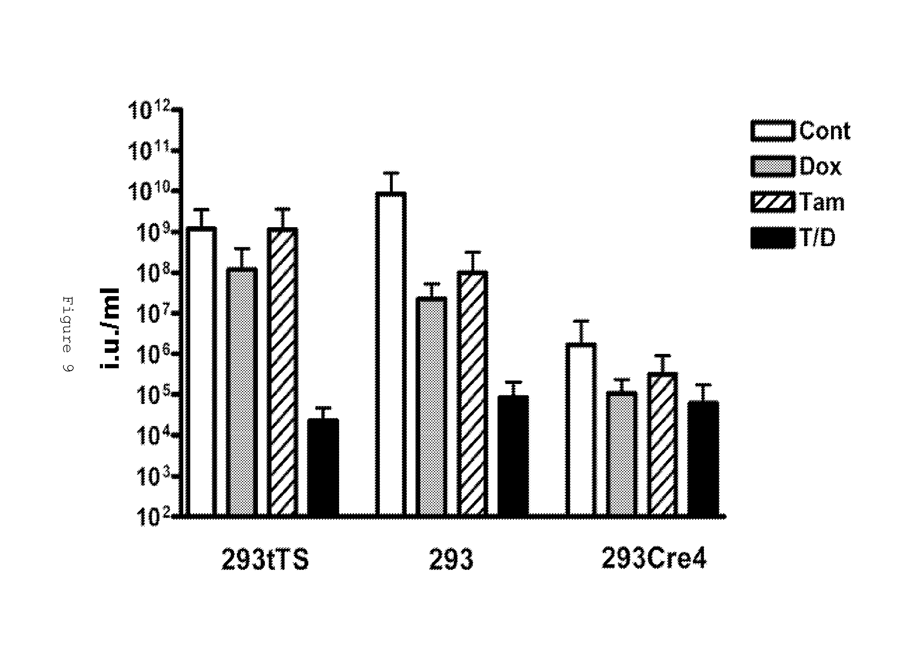

Figure 9 shows the regulation of the production of the AdTetCre virus. 293tTs, 293 and 293Cre4 cells were infected with an MOI of 2 viruses/cell of the AdTetCre virus for 48 hours, and the production of infective virus was quantified in the cell extracts. The cells received a prior 24-hour treatment with doxycycline (Dox), tamoxifen (Tam), both at the same time (T/D), or remained untreated for this period (Cont), as indicated. Treatments were maintained during the time of infection.

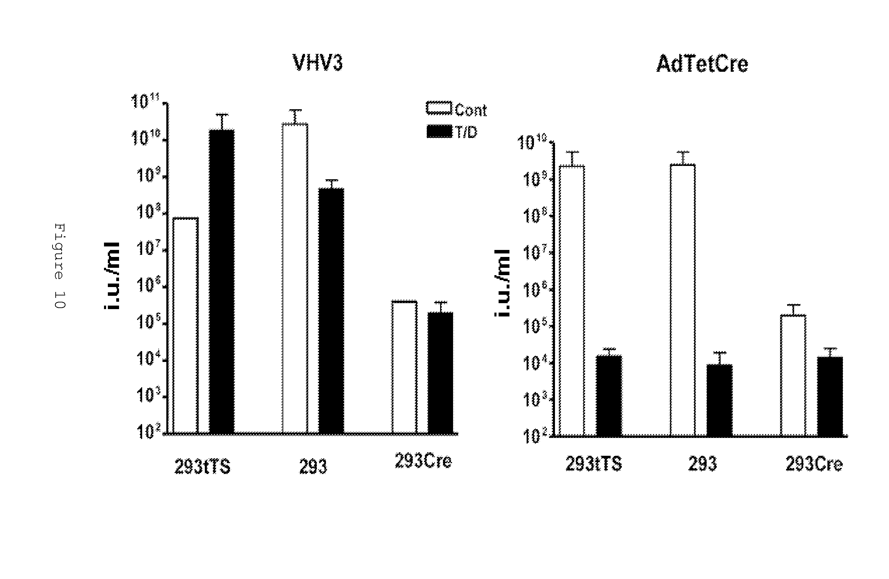

Figure 10 shows the regulation of the production of the AdTetCre virus in comparison with a standard helper adenovirus. 293tTs, 293 and 293Cre4 cells were infected with an MOI of 2 viruses/cell of the AdTetCre or AdLC8cluc virus. The cells received a prior 24-hour treatment with doxycycline and tamoxifen (T/D) or remained untreated for this period (Cont), as indicated. Treatments were maintained during the time of infection. At the end of 48 hours, the production of infective virus was quantified in the cell extracts.

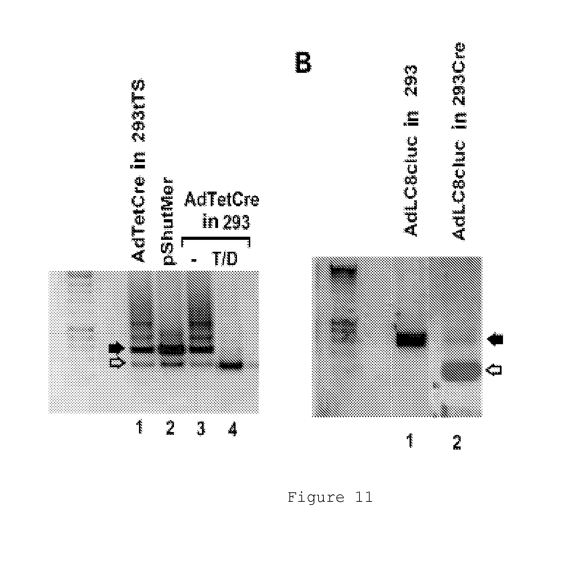

Figure 11 shows the excision of the packaging signal of the AdTetCre and AdLC8cluc virus. (A) 293 cells were infected with an MOI of 2 viruses/cell with the AdTetCre virus for 48 hours. The cells received a prior 24-hour treatment with doxycycline and tamoxifen (line 4) or remained untreated for this period (line 3), as indicated. Treatments were maintained during the time of infection, after which time the DNA of the infected cells was extracted and the area corresponding to the beginning of the viral genome was amplified by PCR. A PCR product using the pShutMer plasmid as a mold, which plasmid contains the same region of the genome (line 2) or the purified AdTetCre virus, produced in 293tTs cells (line 1), is shown as a control. (B) 293Cre4 cells (line 2) were infected with an MOI of 2 viruses/cell with the AdLC8cluc virus and the DNA was extracted 48 hours later. PCR analysis was carried out as in the previous section. The DNA of the purified AdLC8cluc virus, produced in 293 cells (line 1), was used as a control. The size corresponding to a fragment with an intact or excised packaging signal is indicated with a black or white arrow, respectively.

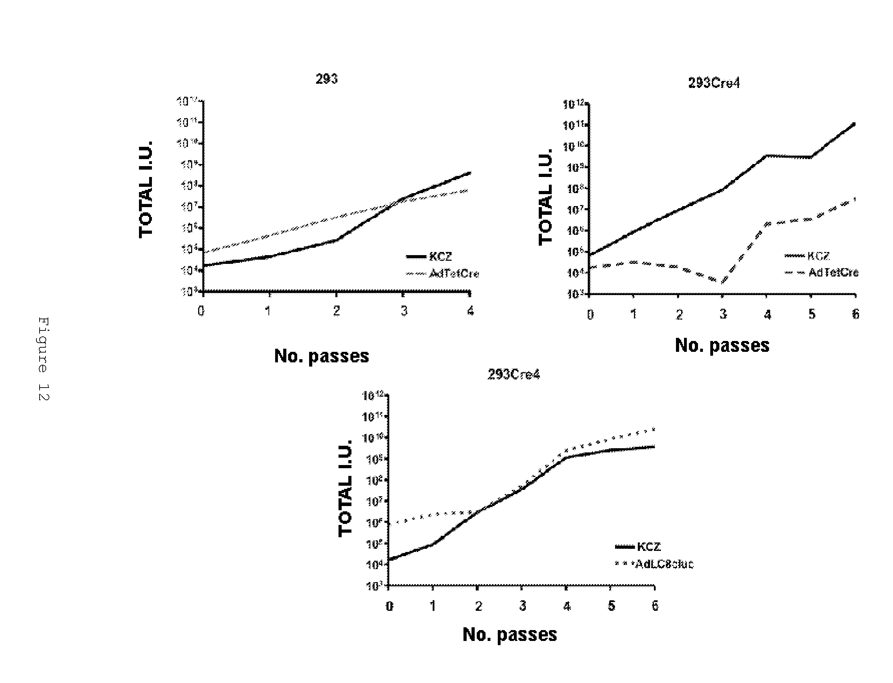

Figure 12 shows the use of the AdTetCre virus in the production of HC-Ad. (A) 293Cre4 cells were transfected with the plasmid containing the genome of HC-Ad KCZ, and 6 hours later they were infected with an MOI 1 of AdTetCre helper virus. The cells were collected after 48 hours (pass 0). The lysate of the previous pass was used in successive passes to infect more 293Cre4 cells together with an MOI 1 of AdTetCre virus. Passes 0-3 are carried out in 60 mm plates. Pass 4 corresponds to 2 150 mm plates, pass 5 are 8 150 mm plates, and pass 6 is equivalent to 30 150 mm plates. The amount of KCZ and AdTetCre viruses was quantified in each pass by means of immunocytochemistry. In all cases the cells were treated with doxycycline and tamoxifen. (B) The same protocol was followed in the 293 cells up to pass 4. (C) The same protocol was followed in 293Cre4 cells using the AdLC8cluc helper virus not treated with doxycycline and tamoxifen.

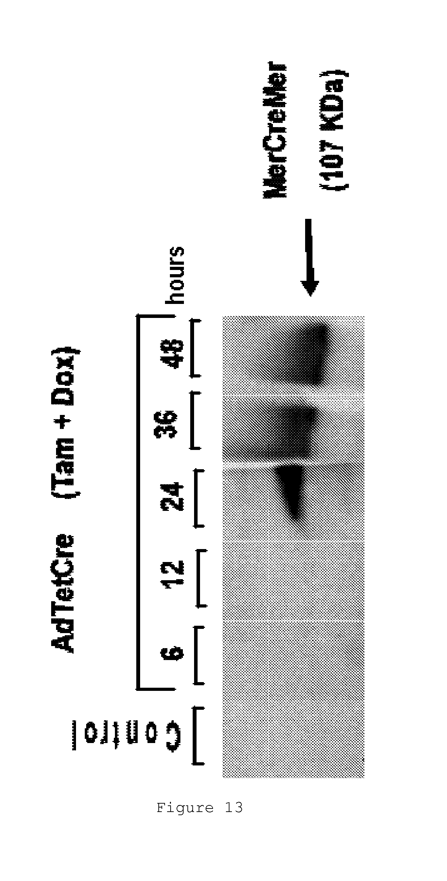

Figure 13 shows the expression kinetics of the MerCreMer protein in the AdTetCre virus. 293 cells were subjected to a 24-hour pre-treatment with tamoxifen and doxycycline, after which they were infected with the AdTetCre virus at an MOI of 2 viruses/cell. After 6, 12, 36 and 48 hours, the cells were collected and the expression of MerCreMer was analyzed by means of immunoblotting. An extract of non-infected 293 cells was loaded in the control line.

### Detailed Description of the Invention

The inventors have shown that helper adenoviral vectors in which the packaging sequence is flanked by recognition target sites of a recombinase and which comprise additionally a sequence encoding a recombinase, wherein said sequence is operatively associated to an inducible promoter, surprisingly allow the generation of gutless adenovirus with a low degree of contamination with the helper viruses. With no intention of being related to any theory, it is thought that the inclusion of the coding sequence in the actual helper adenovirus allows the expression of the recombinase to increase in parallel to the increase of copies of the actual virus, such that there is always a sufficient amount of recombinase to excise the packaging sequence of all the copies of the genome of the helper adenovirus and it is always insensitive to the inhibition of the expression of cellular proteins caused by the actual virus. Figure 1 shows comparative schemes of conventional adenovirus packaging methods in which the recombinase is expressed by the cell in which said packaging (left panel) is carried out or according to the method of the invention in which the recombinase is expressed by the genome of the actual helper virus (right panel). The experimental part describes in an illustrative manner (see example 3) that the adenoviruses comprising the features of the invention express recombinase dependent on the presence of doxycycline in the medium.

### Polynucleotide of the invention

Therefore, in a first aspect, the invention relates to a polynucleotide comprising:
(i) a region encoding a site-specific recombinase, wherein said region is under operative control of an inducible promoter and
(ii) an essential region for the replication and/or for the packaging of the adenoviral genome flanked by recognition sequences specific for the recombinase encoded by sequence a), wherein said recognition sequences are oriented such that the excision of said essential region occurs in the presence of said recombinase.

According to the invention, site-specific recombinase is understood as a protein which is capable of promoting the site-specific recombination between target sites for said recombinase. Recombinases suitable for their use in the invention include Cre recombinase of P1 phage (LoxP), FLP recombinase of *S. cerevisiae* (Frt), integrase from the Streptomyces ΦC31 phage (attB, attP), TP901-1 recombinase, R4 recombinase, or lambda integrase. In a preferred embodiment, the recombinase encoded by the polynucleotide of the invention is the Cre recombinase which includes both the Cre recombinase of P1 phage, as originally described by Abreinski and Hoess (J. Biol. Chem. 1984, 259:1509- 1514), as well as fusion proteins comprising the sequence of the recombinase and a second polypeptide regulating the activity of the recombinase. The Cre recombinase, both if it is used as it is and if it is used in the form of a fusion protein, can be modified so that it has a broader specificity with regard to target sequences as it has been described in WO2000060091

In the event that the polynucleotide of the invention comprises a sequence encoding a fusion protein comprising a recombinase, the use of polypeptides acting as activatable nuclear localization sequences, i.e., they only act as such in the presence of a certain ligand, is possible. It is thus possible to freely regulate the activity of the recombinase such that it translocates to the nucleus and exercises its activity only in the presence of said ligand. In a preferred embodiment, the activatable nuclear localization sequence comes from a nuclear receptor. Nuclear localization sequences suitable for use in the invention include the localization sequence derived from PPAR (peroxisomal proliferator-activated receptors), which translocate to the nucleus in the presence of 15-deoxy-[Delta]-prostaglandin J2, retinoic acid receptors which translocate to the nucleus in the presence of alpha, beta or gamma isomers of 9-cis-retinoic acid, farnesoid X receptors, activatable by retinoic acid and TTNPB, hepatic X receptors activatable by 24-hydroxycholesterol, benzoate X receptors, activatable by 4-aminobutyl benzoate, constitutive androstane receptor, pregnane receptors, inducible by pregnolone-16 carbonitrile and steroid and xenobiotic receptors, inducible by rifampicin, progesterone receptors, inducible by medroxyprogesterone as well as by mifepristone agonists and antagonists and 19-norethisterone derivatives, glucocorticoid receptors activatable by glucocorticoids, thyroidal hormone receptors, activatable by T3 and/or T4 and estrogen receptors, activatable by estrogens and their derivatives such as 17 beta-estradiol and estradiol. Alternatively, it is possible to use a fusion protein which is inactivated by means of its binding to another protein and which, in the presence of a ligand, is dissociated from said additional protein, thus resulting in the inducible activation of the fusion protein.

The activatable nuclear localization sequence preferably corresponds to the ligand binding domain of the estrogen receptor, as described in WO0228175 In the context of the invention, "ligand binding domain" is understood as the sequence of the estrogen binding domain of the estrogen receptor and preferably the variants thereof comprising one or more mutations resulting from the insertion, deletion and/or substitution of one or more amino acids but which maintain the capacity of translocating to the nucleus substantially intact in the presence of a certain ligand, although the preferred ligands of the variants can vary with regard to those of the wild-type receptor. Thus, the invention contemplates the use of nuclear localization sequences preferably responding to natural ligands of the estrogen receptor (estradiol, estriol or estrone) or to different agonists and antagonists thereof, such as tamoxifen, ICI 164384, RU 54876, diethylstilbestrol, raloxifene, zuclomiphene and toremifene.

The recombinase and nuclear localization sequence can be found in any relative location with regard to the other element of the fusion protein. Therefore, the invention contemplates polynucleotides encoding fusion proteins in which the C-terminal region of the nuclear localization sequence is connected to the N-terminal region of the recombinase, polynucleotides encoding fusion proteins in which the C-terminal region of the recombinase is connected to the N-terminal region of the nuclear localization sequence and preferably polynucleotides encoding fusion proteins formed by a recombinase flanked by two nuclear localization sequences which can be identical or different, although they are preferably identical. Additionally, the invention contemplates the use of variants of the nuclear localization sequence which show an affinity for the ligands different from that shown by the nuclear localization sequence of the wild-type receptor. Thus, the invention contemplates the use of the sequence of the ligand binding domain of the estrogen receptor comprising the G512R mutation (numbered according to the human estrogen receptor). This mutation results in an affinity of the ligand binding domain to the natural ligand that is 1000 times less than that shown by the ligand binding domain of the wild-type receptor, but which, however, does not affect its affinity for 4-hydroxytamoxifen. It is thus possible to promote the translocation of the fusion with the protein with recombinase activity by using 4-hydroxytamoxifen without affecting the possible response of the packaging cell to the estrogens. Alternatively, the ligand binding domain of the estrogen receptor can contain the G400V/M543A/L544A mutations, which result in a ligand binding domain that is 10 times more sensitive the nuclear translocation of which can be activated using a dose that is 4 times lower than that necessary for activating the nuclear translocation of the G512R mutant. The ligand binding domain can alternatively comprise the M543 and L544A mutations giving rise to a molecule showing a sensitivity to tamoxifen and 4-hydroxytamoxifen that is 10 times greater than that shown by the G400V/M543A/L544A mutant. In a preferred embodiment, the activatable nuclear localization sequence is the ligand binding domain of the human estrogen receptor (Metger et al., 1995, Proc. Natl. Acad. Sci USA, 92:6991-6995) or the G521R variant thereof, as has been described by Feil et al. (Proc. Natl. Acad. Sci. USA, 1996, 93:10887-10890). In another preferred embodiment, the recombinase comprises the G525R variant of the nuclear localization sequence activatable by estrogens derived from the murine estrogen receptor (amino acids 281-599 of said receptor) at each end of the protein, such as, for example, the MerCreMer protein as described in detail by Verrou et al. (Biol. Chem., 1999, 380:1435-1438) and Tannour-Louet M. et al. (Hepatology, 2002; 35; 1072-1081), the contents of which are included herein by reference.

The polynucleotide of the invention comprises a sequence encoding a recombinase and which is under the operative control of an inducible promoter. In the context of the invention, inducible promoter is understood as any DNA sequence which is capable of promoting the transcription of a polynucleotide located in 3' direction in the presence of a compound or certain condition. Preferably, the inducible promoters which can be used in the context of the invention are those responding to an inducing agent, which show a null or insignificant basal expression in the absence of an inducing agent and which are capable of promoting the activation of the gene located in position 3'. Depending on the type of inducing agent, the inducible promoters are classified in Tet on/off promoters (Gossen, M. and H. Bujard (1992) Proc. Natl. Acad. Sci. USA, 89:5547-5551; Gossen, M. et al., 1995, Science 268:1766-1769; Rossi, F.M.V. and H.M. Blau, 1998, Curr. Opin. Biotechnol. 9:451-456); Pip on/off promoters (US 6,287,813); antiprogestin-dependent promoters (US 2004132086), ecdysone-dependent promoters (Christopherson et al., 1992, Proc. Natl. Acad. Sci. USA, 89:6314-6318; No et al., 1996, Proc. Natl. Acad. Sci. USA, 93:3346-3351, Suhr et al., 1998, Proc. Natl. Acad. Sci. USA, 95:7999-8004 and WO9738117), a metallothionein-dependent promoter (WO8604920) and rapamycin-dependent promoters (Rivera et al., 1996, Nat. Med. 2:1028-32).

In a preferred embodiment, the inducible promoter is a tetracycline-dependent system. This system allows obtaining high expression levels in a manner regulated by tetracycline or derivatives thereof, such as doxycycline. In this system, the expression of the gene is activated in the presence of doxycycline and is inactivated in the absence of doxycycline. This system is based on two regulating elements occurring in the tetracycline resistance operon of E.coli, specifically, the tetracycline operating sequence, to which tetracycline repressor binds. The gene of interest is cloned in 3' direction with regard to a promoter comprising several response elements to tetracycline. A second plasmid contains the sequence encoding a regulating element which is referred to as the tetracycline-controlled transactivator, which is made up of the VP16 transcriptional factor of the herpes simplex virus and of the wild-type tetracycline repressor (rtTA-M2, described by Zabala et al., Cancer Res., 64:2799-2804). Therefore, in the presence of doxycycline, the fusion protein formed by the tetracycline repressor and VP16 interact with the inducible promoter by promoting the transcription of the gene located in 3' direction with regard to the promoter. Said inducible promoter is preferably formed by several copies of the Tet operator (TetO) fused to a minimum promoter such as, for example, the minimum promoter of CMV, the promoter of 29 base pairs of the central protein of the human hepatitis B virus or the promoters of region 5' of the mouse albumin gene of 196 or 84 base pairs, or of the albumin promoter (Zabala et al., 2004, *supra*).

The person skilled in the art will understand that the transactivator of response to tetracycline can be encoded by the genome of the cell used for the packaging, by the actual polynucleotide comprising the sequence encoding the recombinase under the control of the promoter sensitive to tetracycline or, in a preferred embodiment, by both. The amount of transactivator produced will thus depend on the number of copies of the adenoviral genome in the cell, which in turn will allow adapting the amount of transactivator to the amount of promoters present in the cells. The sequence encoding the transactivator is regulated by a constitutive promoter. Constitute promoters suitable for regulating the ligand-dependent transactivator are, among others, the CMV promoter, the SV40 promoter, the DHFR promoter, the mouse mammary tumor virus (MMTV) promoter, the elongation factor 1a (EFla) promoter, the albumin promoter, the ApoA1 promoter, the keratin promoter, the CD3 promoter, the immunoglobulin heavy- or light-chain promoter, the neurofilament promoter, the neuron-specific enolase promoter, the L7 promoter, the CD2 promoter, the myosin light-chain kinase promoter, the HOX gene promoter, the thymidine kinase promoter, the RNA Polymerase II promoter, the MyoD gene promoter, the phosphoglycerokinase (PGK) gene promoter, the low-density lipoprotein (LDL) promoter, the actin gene promoter. In a preferred embodiment, the promoter regulating the expression of the transactivator is the PGK gene promoter.

The polynucleotide of the invention also comprises an essential region for the replication and/or for the packaging of the adenovirus which is flanked by target sites for the recombinase which is encoded by said polynucleotide. Regions of the adenoviral genome essential for the replication and/or for the packaging of said genome include the inverted terminal repeats (ITR) and the adenoviral packaging sequence ψ.

According to the invention, inverted terminal repeat or ITR is understood as sequences of approximately 100 base pairs which are on both sides of the linear genome of the adenovirus and which are essential for the replication of the adenoviral genome (Stow, N.D., 1982, Nucl. Acid. Res, 10:5105-5109).

According to the invention, adenoviral packaging signal ψ is understood as a sequence of approximately 160 base pairs long which, in the case of the adenovirus of serotypes 2 and 5, extends between positions 190 and 350 of the genome. The elimination of the sequence of the genome of an adenovirus prevents the DNA molecules which are generated during the multiplication of the virus from being efficiently incorporated to the recently formed capsids (Hearing, P. et al., 1987, J. Virol., 61:2555-2558), but they do not prevent the replication of said genome in the packaging cell, unlike the elimination of ITRs.

In a preferred embodiment, the region of the adenoviral genome forming part of the polynucleotide of the invention is the adenoviral packaging signal. Obviously, the corresponding target sites flanking the adenoviral packaging signal must be chosen based on the recombinase which is encoded by the polynucleotide of the invention. Therefore, if the recombinase encoded by the polynucleotide is the Cre recombinase, the packaging sequence must be flanked by LoxP sites. If the recombinase encoded by the polynucleotide is the FLP recombinase, the packaging sequence must be flanked by FRT sites. In a preferred embodiment, the recombinase encoded by the polynucleotide of the invention is the Cre recombinase and the target sites of said recombinase (LoxP sites) are flanking the packaging sequence ψ in the same direction such that an excision of the sequence comprised between them occurs.

The LoxP sites are formed by a sequence of 34 base pairs which are in turn formed by two inverted repeats of 13 base pairs flanking a central region of 8 base pairs. The invention also contemplates the use of variants of the wild-type LoxP sequence which are incapable of undergoing recombination with the LoxP sequences, but which can only recombine with identical LoxP variants, such as those described in WO19992585 and WO2007085906 These variant LoxP sequences are particularly useful when the cell which is going to be used for the packaging of the adenovirus comprises regions in its genome flanked by wild-type LoxP sequences. Variant LoxP sequences useful in the context of the invention include the loxP, loxP2, loxP511, loxP514, loxB, loxC2, loxL, loxR, loxA86, loxA117, loxP3 and loxP23 sequences.

### Helper adenoviral vectors of the invention

The previously described polynucleotides of the invention can form part of or can be useful for the construction of an expression vector including sequences necessary for its function as an adenoviral vector. Therefore, in another aspect the invention relates to an expression vector comprising a polynucleotide of the invention. In a preferred embodiment, the vector comprising the polynucleotide of the invention is a helper adenoviral vector.

In the context of the invention, "helper vector" is understood as a vector capable of partially or completely trans-complementing a recombinant viral vector which is incapable of being replicated. It is therefore a vector that is capable of producing at least one polypeptide, early or late, which the recombinant vector is incapable of producing and which is necessary for the formation of the viral particle. The helper vectors can completely complement the adenoviral vector, i.e., the helper vector is capable of providing all those components of the adenovirus necessary for the replication which the adenoviral vector lacks, or it can only partially complement the adenoviral vector, i.e., it is capable of complementing only part of the functions which the adenoviral vector lacks.

The person skilled in the art will understand that the sequence of the helper adenoviral vector will depend on the adenoviral vector which is used given that, depending on the number of coding sequences of the adenoviral genome which are absent in the adenoviral vector, the helper vector must include a higher or lower number of sequences to allow the replication and packaging of said adenoviral vector. In the event that the adenoviral vector is a gutless vector (also known as a high-capacity adenoviral vector or helper-dependent vector), the helper vector of the invention must include virtually all the early and late adenoviral transcription regions, with the exception of early region E3, which is not necessary for in vitro viral replication, and preferably region E1 also, which can be provided by the packaging cell. In a preferred embodiment, the helper vector of the invention comprises the genome of a first-generation adenoviral vector, **characterized in that** it lacks the coding sequences for the E1 and E3 proteins and wherein the gene encoding the recombinase is inserted instead of the region encoding for the E1 protein. The helper adenoviral vector of the invention thus comprises the following elements:
- The 5' inverted terminal repeat,
- The packaging sequence ψ flanked by recognition target sites for a recombinase,
- The sequence of the recombinase, under the control of an inducible promoter,
- The adenovirus coding sequences, except the E1 and E3 regions.
- The 3' inverted terminal repeat.

In a preferred embodiment, the genome of the helper adenovirus encodes a transactivator which is capable of promoting the expression of the recombinase in the presence of a ligand. In an even more preferred embodiment, the transactivator is the rtTA-M2 transactivator, formed by the tetracycline repressor bound to the VP16 protein of the herpes simplex virus, in which case step (iii) of the method is carried out in the presence of a tetracycline analog, preferably doxycycline.

Figure 2 provides a diagrammatic representation of the adenoviral vector of the invention including the sequence encoding the transcriptional transactivator under the control of the PGK gene promoter, and wherein the recombinase is under the control of a promoter formed by several repeats of the tet operator associated to the minimum promoter of the albumin gene.

The packaging sequence ψ is preferably flanked by LoxP sites. In an even more preferred embodiment, the recombinase is Cre recombinase or MerCreMer recombinase which can be under the control of a promoter formed by a series of repeats of the TetO operator and by a minimum promoter such as, for example, the albumin promoter. It is also possible to use attenuated packaging sequences also flanked by recombinase target sites. These signals show a lower capacity of being encapsidated and can be obtained, for example, by means of the deletion of the packaging region as described in WO9428152

It is also possible to increase the safety of the helper vector by means of the use of a pair of recognition target sites for additionally recombinases flanking another essential region of the genome of the said adenovirus, such as for example, ITR regions, such that the presence of the recombinase causes the elimination of more than one region of the genome of the adenovirus which is indispensable for its replication or encapsidation.

The person skilled in the art will understand that the helper vector can be a replicative virus, i.e., it has all the genes necessary for its replication and packaging, or it can be a defective adenovirus in one or several genes, for the purpose of preventing the possible propagation of the adenoviral vector in the surrounding area. Given that these proteins are essential for the propagation of the adenovirus, they must be provided in *trans,* preferably by the cells used for the packaging. Cell lines suitable for this purpose are the 293 cell line (Graham et al., 1977, J. Gene. Virol. 36, 59-72), derived from human embryonic kidney cells and capable of complementing the E1 function given that they contain the 5'-terminal end of the type-5 adenovirus integrated in its genome, the W162 cell line which is capable of complementing E1 and/or E4 functions of the adenovirus (Weinberg et al., Proc. Natl. Acad. Sci. USA, 1983, 80:5383-5386), cell lines capable of complementing the whole genome of the type-5 adenovirus or E1 and E4 functions (WO9428152) N52 line, Perc line and the like.

The helper adenoviral vector of the invention can be found as a polynucleotide comprising all the previously described functions or, alternatively, if the polynucleotide has been generated and/or amplified by means of its transfection in permissive cells, it will give rise to the generation of whole adenoviral particles which can be recovered from the packaging cells and used as helper vectors instead of the polynucleotide. Therefore, in another aspect, the invention relates to an adenoviral particle comprising the sequence of a polynucleotide of the invention. In the context of the present invention, adenoviral particle is understood as a particle formed by a double-strand DNA molecule forming the adenoviral genome, as they have been described, for example, by Shenk, T. (Adenoviridae: The viruses and their replication, 1996, In Fields Virology. B.N. Fields, D.M. Knipe, and P.M. Howley, eds. (Lippincott-Raven, Philadelphia, PA) pp. 2111-2147).

### Systems and methods of propagation of the helper adenoviruses of the invention

The helper vectors of the invention can be amplified by means of successive replication cycles in permissive eukaryotic cells. Therefore, in another aspect, the invention relates to a system for the replication and packaging of an adenovirus recombinant, comprising:
a) a polynucleotide of the invention, an expression vector of the invention or an adenoviral particle of the invention and
b) a eukaryotic cell.

The person skilled in the art will understand that the replication of the adenovirus in the eukaryotic cell requires that expression of the recombinase encoded by the polynucleotide of the invention does not occur, given that if the recombinase is expressed, it would cause the excision of region of the adenoviral genome which is flanked by target sites of said recombinase, which would prevent the propagation of new helper viruses. In the event that the sequence encoding the recombinase is under the control of the tetracycline operator, the latter has a low basal expression level. However, this basal expression can be even further reduced if the cells in which the propagation of the helper adenovirus is carried out express a tetracycline-dependent repressor which is capable of preventing the transcription of genes positioned in 3' direction with regard to the tetracycline operators in the absence of doxycycline. Therefore, in a preferred embodiment, the cell forming the component (b) of the system for the adenovirus replication according to the invention comprises a promoter-specific transcriptional repressor regulating the expression of the site-specific recombinase. In a preferred embodiment, if the promoter regulating the expression of the recombinase is a promoter activatable by tetracycline or an analog thereof (doxycycline), the repressor expressing the packaging cell is a repressor which is activated in the absence of said ligand. Therefore, in a preferred embodiment, the packaging cells of the invention contain a polynucleotide expressing the tetracycline-dependent repressor which, in the absence of tetracycline or doxycycline, binds to Tet operators, blocking transcription. The tet operator-specific repressor is preferably the rtTS repressor. In an even more preferred embodiment, the rtTS repressor is under the operative control of a constitutive promoter. The promoter regulating rtTS expression is preferably the eukaryotic elongation factor EF1a promoter.

The left panel of Figure 3 shows the mechanism allowing repression of the expression of the gene encoding the recombinase in the absence of doxycycline.

Additionally, in the event that the recombinase comprises an activatable nuclear localization sequence, the propagation of the helper adenovirus must be done in the absence of the ligand capable of activating said nuclear localization sequence, such that, even though there is a minimum amount of recombinase due to the basal transcription of said gene, it remains in the cytoplasm and is incapable of exerting its recombinase activity on the adenoviral genome.

The helper vectors of the invention and the packaging systems thereof can be used to amplify said vectors. Therefore, in another aspect the invention relates to a method (hereinafter first method of the invention) for obtaining a helper recombinant adenovirus comprising
(i) contacting a cell with a polynucleotide of the invention, with an expression vector according to the invention or with an adenoviral particle according to the invention and, optionally, with one or several polynucleotides encoding the adenoviral proteins necessary for the packaging and replication of the adenovirus in the cell in suitable conditions so that the entry in the cell of the adenoviral genome or of the polynucleotide comprising said genome occurs,
(ii) keeping the cell in suitable conditions to allow the packaging and replication of the adenovirus in the cell and to prevent the expression of the site-specific recombinase and
(iii) recovering the adenoviruses from the medium.

Step (i) of the first method of the invention comprises introducing the genome of said adenovirus in the cell. The person skilled in the art will understand that step (i) is carried out differently, depending on the form which said adenoviral genome is in. In the event that said genome is found as a polynucleotide, step (i) comprises putting said polynucleotide in contact with the cell in conditions which allow its entry in the cell. The transfection can be carried out by means of microinjection of a composition comprising the polynucleotide or, alternatively, by means of co-precipitation in the presence of calcium phosphate or calcium chloride, by means of transfection mediated by DEAE-dextran, lipofection, electroporation, as well as a series of commercially available transfection kits based on the preceding techniques. The efficacy of the transfection will depend on a series of factors including the type of cell, number of passes, state of confluence, as well as the transfection conditions (time, form of preparing the liposomes or the precipitates, etc.). All these parameters can be determined and adjusted by means of routine experimenting.

In the event that the adenoviral vector is an adenoviral particle according to the invention, it is enough to put the adenovirus in contact with the cell so that the adenovirus infects the cell naturally. The virus is preferably put in contact with the cells using a number of viral particles to cell ratio (multiplicity of infection or MOI) of at least approximately 1, 2, 10, 20, 30, 50, 100, 200, 2000 or any other suitable MOI value so that all the cells of the culture are substantially infected.

In the event that the helper adenoviral vector lacks any of the sequences encoding components essential for its replication and/or packaging, it is necessary to provide said components in *trans.* The components are preferably provided from the expression of polynucleotides forming part of the genome of the cells which are used as packagers. Therefore, in the case of the adenoviral vectors the genomes of which lack the E1 and/or E4 regions, the cells which are used in step (i) of the method of the invention are, among others, cells belonging to 293 (Graham et al., 1977, J. Gene. Virol. 36, 59-72) and W162 (Weinberg et al., Proc. Natl. Acad. Sci. USA, 1983, 80:5383-5386) cell lines and the cell lines described in WO942815 as previously described.

In step (ii), the first method of the invention contemplates keeping the cells which have incorporated the adenoviral genome in step (i) in suitable conditions to allow the packaging and replication of the adenovirus in the cell and to prevent the expression of the site-specific recombinase. Given that the recombinase is under the operative control of an inducible promoter, in ideal conditions said promoter should not activate the transcription unless it is in the presence of the activating agent. However, all the inducible promoters have certain basal activity which, in the case of the helper vectors at hand, would give rise to the elimination of the essential sequence (preferably packaging sequence ψ) thus giving rise to adenoviral genomes incapable of being encapsidated and, therefore, reducing the yield of the process. Therefore, in a preferred embodiment, the encapsidation process is carried out in cells expressing a transcriptional repressor which binds to the binding site of the promoter regulating the expression of the recombinase by blocking transcription based on the latter. Said transcriptional repressor is preferably active in the absence of the ligand which activates said transcriptional promoter. In the particular case of a promoter activated by the rtTA tetracycline-dependent activator, the transcriptional repressor binds specifically to tetracycline operators in the absence of doxycycline, therefore blocking the expression of the gene of the recombinase.

Step (ii) is carried out for the necessary time so that the concentración of viral particles is high enough, as determined by standard means such as the Resource Q method based on HPLC, as described by Shabram et al., (Hum. Gene Ther. 1997, 8:453-465), or indirectly by visual inspection of the cytopathic effect in the cells. The cell culture can optionally include the addition of fresh culture medium.

In a third step, the adenoviruses generated during step (ii) are recovered from the cell culture. To that end it will be necessary to separate the cells from the culture medium by any method known by persons skilled in the art (trypsinization or mechanical scraping, and centrifugation or filtration), rupturing the cells in inert solution (freezing/thawing cycles, homogenization, sonication, cavitation, use of detergents and the like) and purifying the adenovirus particles by known methods, such as ultracentrifugation in CsCl gradient, or by means of different types of chromatography, such as a combination of ion exchange chromatography and affinity chromatography with metal chelates (Huyghe et al., 1996, Human Gene Therapy, 6:1403-1416), a combination of ion exchange chromatography and fractional chromatography in gel (WO07059473) chromatography in diethylaminoethyl (DEAE) cellulose (Haruna et al., 1961, Virology 13, 264-267), chromatography in hydroxylapatite (US2002/0064860) and chromatography in other resins, such as soft gels (agarose), macroporous polymers based on synthetic polymers including permission chromatography (fractogel) and materials based on a soft gel in a hard capsule (for example, Ceramica HyperD® F) (Rodrigues, 1997, J. Chromatogr. B Biomed. Sci. Appl 699:47-61).

In another aspect, the invention contemplates the helper adenoviruses obtained according to the first method of the invention.

### Systems and methods of propagation of adenovirus comprising a gene of interest using the helper adenoviruses of the invention

The helper adenoviruses object of the invention can be used in systems and processes for the replication and packaging of high-capacity adenoviruses comprising a gene of interest. Therefore, in one aspect the invention relates to the use of a polynucleotide of the invention or of a vector of the invention or of an adenoviral particle according to the invention for the production and packaging of high-capacity adenoviruses.

In another aspect, the invention relates to a system for the production of a high-capacity recombinant adenovirus comprising
(a) a polynucleotide of the invention, an expression vector according to the invention or an adenoviral particle according to the invention;
(b) an adenovirus the genome of which comprises a gene of interest and which lacks the sequence encoding at least one of the essential proteins for the replication or at least one of the essential proteins for the packaging of said adenovirus or with a polynucleotide comprising the genome of said adenovirus and
(c) a eukaryotic cell.

In said system, the first component is essentially that described in the amplification systems of helper adenoviruses and consists of a polynucleotide comprising at least part of the genome of the helper adenovirus providing either completely or partially the components necessary for the replication and packaging of the high-capacity adenovirus.

The second component comprises high-capacity adenoviruses or polynucleotides comprising the genome of said virus wherein said genome lacks the sequence encoding at least one of the essential proteins for the replication or at least one of the essential proteins for the packaging of said adenovirus and which is going to be amplified using the system of the invention. According to the invention, "high-capacity adenoviruses" is understood as recombinant adenoviruses of they type referred to as helper-dependent or gutless adenoviruses **characterized in that** all the genes of the adenoviral genome have been eliminated except the 5' and 3' ITR regions and the packaging region ψ, as has been described, for example, by Alba et al (Gene Therapy, 2005, 12:S18-S27). These adenoviruses allow heterologous sequences of up to 36 kDa given that they lack characteristic sequences of the adenoviral genome.

Heterologous genes that can be incorporated in the gutless vectors according to the methods of the invention include the genes encoding erythropoietin (EPO), leptins, corticotropin-releasing hormone (CRH), growth hormone-releasing hormone (GHRH), gonadotropin-releasing hormone (GnRH), thyrotropin-releasing hormone (TRH), prolactin-releasing hormone (PRH), melatonin-releasing hormone (MRH), prolactin-inhibiting hormone (PIH), somatostatin, adrenocorticotropic hormone (ACTH), somatotropin or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, parathyroid hormone, gastrin, cholecystokinin (CCK), secretin, type I insulin-like growth factor (IGF-I), type II insulin-like growth factor (IGF-II), atrial natriuretic peptide (ANP), human chorionic gonadotropin (hCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), interleukin 2 (IL-2), tumor necrosis factor-alpha (TNF-α), interleukin 6 (IL-6), Interleukin 8 (IL-8 and chemokins), interleukin 12 (IL-12), interleukin 16 (IL-16), interleukin 15 (IL-15), interleukin 24 (IL-24), interferons -alpha, -beta, - gamma, CD3, ICAM-1, LFA-1, LFA-3, chemokins including RANTES 1α, MIP-1a, MIP-1β, neuronal growth factor (NGF), platelet-derived growth factor (PDGF), transforming growth factor-beta (TGF-beta), bone morphogenic proteins (BMPs), fibroblast growth factors (FGF and KGF), epidermal growth factor (EGF and the like), vascular endothelial growth factor (VEGF), granulocyte colony stimulating factor (G-CSF), glial growth factor, keratinocyte growth factor, endothelial growth factor, alpha-1 antitrypsin, tumor necrosis factor, granulocyte and microphage colony stimulating factor (GM-CSF), cardiotrophin 1 (CT-1), oncostatin M (OSM), amphiregulin (AR), cyclosporine, fibrinogen, lactoferrin, tissue-type plasminogen activator (tPA), chymotrypsin, immunoglobulins, hirudine, dismutase superoxide, imiglucerase, β-glucocerebrosidase, α-L-glycosidase-α, α-L-iduronidase, iduronate-2-sulfatase, galsulfase, human α-galactosidase A, α-1 proteinase inhibitor, lactase, pancreatic enzymes (lipase, amylase, protease), adenosine deaminase, immunoglobulins, albumin, type A and B botulinum toxins, collagenase, human deoxyribonuclease I, hyaluronidase, papain, L-asparaginase, lepirudin, streptokinase, beta cell transformation factor (TGF-β) inhibitor peptides such as those described in WO0331155 WO20051924 and WO0393293 the content of which is incorporated herein by reference, expression cassettes suitable for interference RNA molecule transcription (shRNA, siRNA, miRNA, modified U1 ribonucleoprotein RNA).

In addition to said heterologous genes, the gutless adenoviruses used in the invention can contain stuffer regions such as the stuffer sequences derived from the lambda phage genome described by Parks et al. (J. Virol., 1999, 73:8027-8034) or the stuffer DNA appearing in pSTK120 as has been described by Sandig et al., Proc. Natl. Acad. Sci. USA, 97:1002-7). These regions usually contain sequences present in the human genome and capable of increasing the expression of heterologous genes with regard to the control adenoviruses which, instead of said human stuffer DNA, comprise the 22 kb fragment of the lambda phage described in Parks et al. (J. Virol., 1999, 73:8027-8034).

The total length of the human stuffer DNA can vary, but it ranges between 20 to 30 kb, preferably from 20 to 25 kb or, even more preferably, from 21 to 23 kb, for example, 22 kb. The number of sequences acting in cis in said stuffer DNA can vary among 2, 3 or 4 or more and include, without limitation, the 16.2 kb alphoid repeat domain (described in Smith J G et al., 1995, Mol. Cell. Biol., 15:5165-72); matrix attachment regions (MAR), such as for example the 4.2 kb fragment containing the left end of the type 6 adenovirus 6 or two copies of the matrix attachment region of immunoglobulin κ (as described by Betz A G et al., 1994, Cell, 77:239-248); a hepatocyte control region (HCR), such as the 1.2 kb fragment comprising said HCR (as described by Miao et al., Molecular Therapy, 1:522-32); or another matrix attachment region such as the 2.8 kb region of the chicken lysozyme (as described by Phi-Van Let al., 1990, Mol. Cell. Biol. 10:2302-2307, matrix attachment region of the 5' region of interferon β (as described by Bode J et al. 1992, Science, 10:195-197) or other non-encoding human sequences such as telomeric or centromeric sequences.

Finally, the system for the production of a high-capacity recombinant adenovirus according to the invention additionally comprises a eukaryotic cell in which the amplification process of said adenoviruses is carried out. Eukaryotic cells suitable for their use in said system include all those cell lines which can be spontaneously infected by the adenovirus (if the adenoviruses forming components (a) and (b) of the system) or cell lines susceptible of being transfected with polynucleotides comprising the genomes of the high-capacity and helper adenoviruses. Additionally, if the genome of the helper adenovirus does not comprise the sequence encoding the transactivator capable of activating the expression of the recombinase, the cells forming component (c) must express said transactivator, preferably in a stable manner and preferably under the control of a constitutive promoter. This can be beneficial for accelerating the expression kinetics of the recombinase comprised in the helper adenovirus, and thus facilitating the excision of its packaging sequence. In a preferred embodiment, it is possible to use cells expressing the recombinase the target sites of which are flanking the essential region of the genome of the helper adenovirus, such that, in addition to the recombinase produced by the actual helper adenovirus, there is an additional amount of recombinase produced by the actual cell such that greater efficacy is obtained in the elimination of all the replicative genomes of the helper adenoviruses. The invention preferably contemplates the use of established cell lines expressing Cre recombinase in a stable manner, such as the 293Cre line (Parks et al., *supra.).* The recombinase expressing the cell can be under the control of a constitutive promoter or it can be under the control of an inducible promoter which can be the same or different than the one used to regulate the expression of the recombinase encoded by the helper adenovirus. The constitutive and inducible promoters which can be used to regulate the expression of the recombinase encoded by the cell are the same as those mentioned above.

In another aspect, the invention relates to a method (hereinafter second method of the invention) for generating a high-capacity adenoviral vector expressing a gene of interest comprising the steps of
(i) contacting a cell with an adenovirus the genome of which comprises a gene of interest and which lacks the sequence encoding at least one of the essential proteins for the replication or for the packaging of said adenovirus or with a polynucleotide comprising the genome of said adenovirus in suitable conditions so that the entry in the cell of the genome of said adenovirus or of the polynucleotide comprising said genome occurs,
(ii) contacting said cell with a second component selected from the group of:
   (a) a polynucleotide comprising:
      1. a region encoding a site-specific recombinase, wherein said region is under operative control of an inducible promoter and
      2. an adenoviral packaging signal ψ flanked by recognition sequences specific for the recombinase encoded by sequence a) wherein said recognition sequences are oriented such that the excision of the packaging signal ψ occurs in the presence of said recombinase,
   (b) an adenoviral vector comprising a polynucleotide according to (a)
   (c)an adenoviral particle comprising a polynucleotide according to (a)
      in suitable conditions so that the entry in the cell of said polynucleotide, of said adenoviral vector or of the genome of said adenoviral particle occurs,
(iii) keeping the cell in suitable conditions for the expression of the proteins encoded by the genomes of both viruses, for the replication of the genomes of both adenoviruses and for the expression of the recombinase encoded by the polynucleotide, vector or adenovirus used in step (ii) and
(iv) recovering the high-capacity adenovirus from the cell culture.

Step (i) of the second method of the invention is similar to the first step of the first method of the invention, given that it consists of putting a cell in contact with an adenoviral genome, wherein said adenoviral genome can be in form of a nude polynucleotide or forming part of an adenoviral particle. In the first case, step (i) of the method involves the use of known methods so that the polynucleotide accesses the cell interior as has been previously described. In the second case, it is enough to put the adenoviral particle in contact with the cell and, if this expresses the surface molecules acting as receptors for the adenoviruses, the latter will spontaneously incorporate their genome in the cell. In a preferred embodiment, the adenovirus which is used in step (i) is a high-capacity or gutless adenovirus.

Step (ii) of the second method of the invention can be carried out simultaneously, before or after step (i), although it should be carried out after to prevent the cytopathic effect caused by the expression of the structural and regulating proteins of the adenovirus from preventing the efficient replication of the genome of the adenovirus comprising a gene of interest. The entry in the cell of the genome of the helper adenovirus takes place either by means of known transfection techniques if said genome is found as a nude polynucleotide or by following the natural infection route of the adenovirus if said genome is provided forming an adenoviral particle.

Step (iii) of the second method of the invention comprises keeping the cells in suitable conditions to allow the expression by means of the cellular transcription/translation machinery of the structural proteins encoded by the genome of the helper adenovirus which have been introduced in step (ii) and to allow the replication of the genome of the adenovirus comprising the gene of interest. In this step it is essential to assure the expression and activity in the cell of the recombinase encoded by the genome of the helper adenovirus, such that the excision of the region of said genome essential for its replication and/or encapsidation occurs and the formation of adenoviral particles comprising the genome of the helper adenovirus is maximally reduced. In a preferred embodiment, step (iii) is carried out in the presence of a specific transactivator inducer of the promoter regulating the expression of the site-specific recombinase. Said transactivator can be provided by the actual cell in which the packaging process is carried out if the polynucleotide which encodes it is integrated in the genome of said cell in a stable manner or, alternatively, in a preferred embodiment, it is the actual genome of the helper adenovirus which encodes for said transactivator. In an even more preferred embodiment, the transactivator is expressed from the genome of the helper adenovirus and the packaging cell. In an even more preferred embodiment, said transactivator requires the presence of a ligand to exert its transactivating activity, in which case step (iii) of the method is carried out in the presence of said ligand to assure maximum expression of the recombinase. In an even more preferred embodiment, the transactivator is the rtTA-M2 transactivator, formed by the tetracycline repressor bound to the VP16 protein of the herpes simplex virus, in which case step (iii) of the method is carried out in the presence of a tetracycline analog, preferably doxycycline.

The right panel of Figure 3 shows the mechanism by means of which the activation of the expression of the recombinase in the presence of doxycycline occurs.

As previously described, any recombinase is essentially suitable for the elimination of the packaging signal in the helper virus provided that said packaging signal is flanked by the target sites of said recombinase in the suitable orientation to cause the excision of the sequence comprised between both signals. In a preferred embodiment, the recombinase encoded by the helper adenovirus is Cre recombinase or a fusion protein comprising said recombinase. An additional way to assure that the recombinase is inactive when it is not used in the packaging process of the adenovirus of interest is the use of recombinase the translocation of which to the nucleus depends on the presence of a ligand which can be added exogenously, and which binds to cellular proteins preventing its function in the absence of a specific ligand. Therefore, in a preferred embodiment the invention contemplates the use of a fusion protein formed by Cre recombinase and at least one ligand binding region of the estrogen receptor. These types of fusion proteins are located by default in the cytoplasm and bind to certain cellular proteins, and are therefore incapable of exerting their recombinase function. However, in the presence of an estrogen or a derivative (for example tamoxifen), the fusion protein is released from the bond to said cellular proteins and translocates to the nucleus where it exerts its recombinase function. In an even more preferred embodiment, the recombinase encoded by the genome of the recombinant adenovirus is the MerCreMer recombinase, formed by Cre recombinase with a mutated ligand binding region of the estrogen receptor in the N-terminal position and in the C-terminal position.

Figure 6 diagrammatically shows the mechanism by means of which expression and activation of the recombinase in the presence of doxycycline and tamoxifen occurs.

Step (iv) of the second method of the invention comprises the purification of the gutless adenoviruses formed during step (iii) and is essentially carried out in the same manner that helper adenoviruses are purified in step (iii) of the first method of the invention, as described in detail above.

The person skilled in the art will understand that the adenoviruses comprising a gene of interest purified according to the second method of the invention can be used in successive rounds of propagation by means of putting said adenovirus in contact again with a cell, followed by putting said cell in contact with another dose of helper adenoviruses obtained by means of the first method of the invention. The process of the invention can thus be repeated successive times, obtaining a higher number of adenoviral particles in each cycle.

The invention is illustrated below based on the following examples provided in an illustrative and non-limiting manner of the scope of the invention.

### EXAMPLES

### EXAMPLE 1

### Construction of the AdTetCre virus

Construction of the inducible MerCreMer expression cassette.

The inducible system consists of two independent transcriptional units. One of them encodes for the recombinase under the control of an inducible promoter, and the other one encodes for a transactivator under the control of a constitutive promoter (promoter of the murine PGK gene). To obtain the first unit, the previously described pO₇Pal-luc plasmid (Zabala et al., 2004, Cancer Res. 64:2799-2804) was used to start from. This plasmid contains 7 binding sites for the transactivator and a 196 bp region of the 5' end of the of murine albumin promoter controlling the expression of the luciferase gene, and a polyadenylation signal from SV40 virus. The coding sequence for the MerCreMer fusion protein from the pANMerCreMer plasmid (Dr. M. Reth, Max-Planck Institute for Immunobiology, Freiburg, Germany) (Verrou et al., 1999, Biol Chem. 380:1435-8) was introduced in this plasmid. Cloning was carried out using the HindIII restriction sites of the pANMerCreMer plasmid and the HindIII and XbaI sites of the pO₇Pal-luc plasmid, giving rise to the palbMerCreMer plasmid. The promoter of the murine phosphoglycerate kinase (PGK) gene was used to obtain the second unit controlling expression of the rtTA2S-M2 transactivator (Zabala et al., 2004, *supra.),* and a synthetic polyadenylation signal based on the mouse beta-globin gene (Levitt et al., 1989, Genes Dev. 3:1019-25). The resulting plasmid was called pGKrtTA. Both transcriptional units were fused in a single plasmid called prtTAMerCre by means of subcloning a XhoI-SalI fragment of the palbMerCreMer plasmid into the pGKrtTA plasmid.

### -Construction of the genome of the AdTetCre vector.

First a sequence comprising the left-end adenoviral ITR and the packaging signal flanked by LoxP sites was obtained by means of PCR. The pGS102 plasmid, containing the genome of a helper adenovirus (E. Ayuso, Doctoral Thesis. School of Veterinary Medicine. Universidad Autonoma de Barcelona. CBATEG. 2006) was used as a mold for that purpose. The oligonucleotides used were:

| Oligonucleotide | Sequence | SEQ ID NO |
|---|---|---|
| 5Ad5St | TAGTGTGGCGGAAGTGTGATGTTG | 1 |
| AdpTGC-10 | TGACTAGTATAAGCGGCCGCAGCAGATACGGGATATC | 2 |

This fragment was introduced in the pShuttle plasmid (He et al., 1998) to give rise to the pShut-VH plasmid. Then the orientation of the bacterial sequences included in the plasmid (kanamycin resistance and origin of replication) was changed by means of digestion with the PacI enzyme and re-ligation of the fragments. The resulting plasmid was called pShutLoxP. Then a single restriction site for the SwaI enzyme was included between the NotI and SalI positions of the plasmid. To that end a cassette was constructed by means of hybridization of the oligonucleotides

| Oligonucleotide | Sequence | SEQ ID NO |
|---|---|---|
| 5SwaB | CTGACTGAGCGGCCGCATTTAAATGTCGACACTTCAC | 3 |
| 3SwaB | GTGAAGTGTCGACATTTAAATGCGGCCGCTCAGTCAG | 4 |

it was digested with the NotI and SalI enzymes and ligated into the pShutLoxP plasmid. The resulting plasmid was called pShutSwa. Then this plasmid was used as a mold to amplify the fragment containing the left part of the adenoviral genome (including the encapsidation sequence flanked by LoxP sites and the SwaI recognition site). The oligonucleotides used were

| Oligonucleotide | Sequence | SEQ ID NO |
|---|---|---|
| 5ClaITR | ACTGACATCGATTTAATTAACATCATCAATAATATACC | 5 |
| 3ClaLoxP | TAGATTATCGATTCTTAACCACGCCCAGATC | 6 |

This fragment was introduced in the ClaI site of the pAdEasyl plasmid (He et al., 1998, Proc. Natl. Acad. Sci. USA, 95:2509-14). The pAdLoxP2 plasmid, containing the genome of a first generation adenoviral vector (with deletion of the E1 and E3 regions) in which the encapsidation signal is flanked by LoxP sites, was thus obtained. The inducible expression cassette of the MerCreMer protein from the prtTAMerCre plasmid was introduced in this plasmid, using the only SwaI site. This new plasmid (pAdLoxPMerCre) should contain the sequence of the new self-inactivatable helper adenovirus, but it was observed that the encapsidation sequence is spontaneously excised during the amplification process of this plasmid in E. Coli, probably by expression of the MerCreMer recombinase. To solve this problem, a strategy based on the homologous recombination in mammal cells was used. To that end, a SnaBI-SapI fragment of the prtTAMerCre plasmid containing the inducible expression cassette, but with the coding sequence for MerCreMer truncated, was introduced in the pShutSwa plasmid. The co-existence of LoxP sites and the capacity of expressing recombinase activity is thus prevented in the same plasmid. The resulting plasmid was called pShutMer. To produce the new helper virus, the pAdLoxPMerCre and pShutMer plasmids were co-transfected in 293tTS cells. These plasmids have ample areas of homology, such that the recombination which may give rise to a functional viral genome is facilitated (Figure 7). When an evident cytopathic effect was observed in the cell cultures (approximately 10-15 days after transfection), the cells were lysed and the structure of the virus was verified by means of PCR and sequencing of the most important areas. Individual clones were obtained by means of limit dilution in 293tTS cells. The new self-inactivatable helper adenovirus was called AdTetCre.

### Amplification, purification and quantification of the virus

The AdTetCre virus was amplified in 293tTS cells. Once the cytopathic effect was observed in most of the cells, they were collected along with the culture medium and centrifuged. The supernatant was discarded and the cells were resuspended again in a smaller volume of 10 mM Tris, pH 8.1. Then they were lysed by means of 3 freezing and thawing cycles, and the released virus was purified by means of a double cesium chloride gradient. The band corresponding to the virus was collected and the salt was eliminated by means of a size exclusion column (Sephadex). The quantification of the virus in ineffective units was carried out by means of the commercial AdenoX rapid titer kit (BD Biosciences), according to the manufacturer's specifications.

### Expression of the MerCreMer protein

293tTS cells were seeded in a 6-well multiwell plate (7.5 x 10⁵ cells/well). After 24 hours they were pretreated with tamoxifen (0.8 µM) and doxycycline (100 ng/ml), or remained untreated. At 48 hours they were infected with the AdTetCre virus at an MOI of 2 viruses/cell, maintaining treatment with tamoxifen and doxycycline during infection. The cells were collected after 48 hours. In the case of the control of 293 cells, they were cultured in a 6-well multiwell plate and collected at the same time as the preceding infections. The same was done for the case of the control of 293Cre4 cells. To study the expression kinetics of the MerCreMer protein, the cells were treated in the same manner and collected at different times (6 h, 12 h, 36 h and 48 h). To obtain a positive control of the MerCreMer protein, 293tTS cells were transfected with the pANMerCreMer plasmid. The transfection was carried out with 20 ul of lipofectamine 2000 and 15 µg of the non-digested plasmid. The cells were collected at 48 hours. In all cases the cells were lysed with a solution made up of TNE buffer (50 mM Tris pH 7.5, 5 mM EDTA, 100 mM NaCl) supplemented with 1% Igepal and a mixture of protease inhibitors (Complete Tablets, Roche) for 15 minutes in ice and were subsequently sonicated and centrifuged at 15,000 g for 45 minutes at 4°C. The amount of protein was determined by means of the Bradford method and then electrophoretic separation of the proteins was carried out by means of SDS-PAGE in 8% in which 20 µg of protein per sample had been loaded. The Cre or MerCreMer protein was detected by means of Western Blotting using a 1:10.000 dilution of the primary antibody specific against Cre (Novagen) and a 1:5.000 dilution of the secondary goat anti-rabbit antibody bound to peroxidase (BioRad). They were developed by means of Western Lightning Chemiluminescence Reagent (Perkin Elmer).

### Control of viral production by means of doxycycline and tamoxifen

The different cells (293, 293tTS or 293Cre4) were seeded in 12-well plates with 4x10⁴ cells per well and were kept in DMEM culture medium supplemented with 10% FBS, Glutamine, penicillin and streptomycin. After 24 hours, the cells were treated with doxycycline (100 ng/ml), tamoxifen (0.8 µM) or both at the same time, or they remained untreated. The infection was carried out 24 hours later with an MOI of 2 viruses/cell of AdTetCre helper virus or AdLC8cluc, and 48 hours later the cells were collected. The quantification of the amount of infective virus was carried out by means of AdenoX rapid titer kit (BD Biosciences), according to the manufacturer's specifications

### HC-Ad production protocol

The pKCZ plasmid, containing the genome of HC-Ad KCZ, was digested with PmeI restriction enzyme in order to linearize it and eliminate resistance to the antibiotic. It was then precipitated by adding sodium acetate (300 mM) and 2.5 volumes of absolute ethanol. The sample was kept at -20 °C for at least 1 hour and was then centrifuged at 11,000 g for 30 minutes at 4 °C. The precipitate was washed with 70% ethanol and air dried. It was resuspended in water and 15 µg were used to transfect 1x10⁶ 293Cre4 cells in a 60 mm plate which had been pretreated for 24 hours with tamoxifen and doxycycline. The transfection was carried out using 20 µl of Lipofectamine 2000 (Invitrogen). The medium was changed at 6 hours, adding DMEN medium with 2% FBS and the AdTetCre virus at an MOI of 1 virus/cell. This MOI was determined in a prior experiment in which the amount of virus necessary to produce 90% of the cytopathic effect at 48 hours of infection was assayed. The infection was left to progress for 48 hours and the cells were then collected in 200 µl of Tris 10 mM, pH 8.1. This transfection-infection step is called pass 0. The virus produced in this phase was released from the cells by means of 3 freezing/thawing cycles and 150 µl of the lysate were used to infect a new plate of 293Cre4 cells along with an MOI 1 of the AdTetCre helper virus. As in the previous case, the cells were pretreated for 24 hours with doxycycline and tamoxifen. The so-called passes 1-3 are thus carried out. In pass 4, 2 150 mm plates are infected with 1 x 10⁷ 293Cre4 cells in each one, in pass 5 there are 8 150 mm plates, and the final pass of the mid-scale production consists of 30 150 mm plates. The produced virus was purified by means of a double cesium chloride gradient and the salt was eliminated from the preparation by means of a size exclusion column (Sephadex). The amount of AdTetCre was quantified in each of the passes by means of the commercial AdenoX rapid titer kit (BD Biosciences), according to the manufacturer's specifications. HC-Ad KCZ was quantified by means of staining with X-Gal.

Production in 293 cells was carried out in the same way, reaching up to pass 4. Production with the AdLC8cluc helper virus was carried out in 293Cre4 cells not treated with doxycycline and tamoxifen, reaching up to pass 6.

### EXAMPLE 2

### Construction of a plasmid system allowing the expression of Cre in a manner regulated by doxycycline and tamoxifen

To achieve control of the expression of Cre, a "tet-on" type inducible system which has been previously optimized (Zabala et al., 2004, *supra.)* was used. The system is based on the constitutive expression of a transactivating protein (rtTA-M2) which dimerizes in the presence of exogenously added doxycycline, adopting an active conformation capable of binding to promoters containing "tet" binding sequences (Figure 2). It is thus achieved that the gene which is under the control of the inducible promoter (with "tet" sites) is expressed only in the presence of doxycycline, in a dose-dependent manner. In this case, a plasmid has been constructed in which the rtTA-M2 transactivator is under the control of a constitutive promoter (phosphoglycerate kinase, PGK), to assure that it can be active in any HC-Ad packaging cell. In addition, Cre is under the inducible promoter formed by several "tet" sites bound to a minimum promoter (in this case the albumin promoter). Then both constructs were fused to obtain a plasmid with the complete inducible expression system of Cre (Figure 2).

To assure that the basal expression of Cre does not prevent the production of the new helper virus, two complementary strategies have been carried out.

On one hand, cells constitutively expressing the tTS repressor have been generated. The tTS protein is capable of binding to the inducible promoter in the absence of doxycycline, and blocking transcription. When the doxycycline inducer is added, tTS changes conformation and no longer belongs to the promoter, leaving an open path for the rtTA-M2 transactivator to bind and activate expression of Cre (Figure 3). Therefore, the method is based on in two types of packaging cells: 293 expresing tTS for the production of the helper adenovirus, and 293 cells (with or without constitutive expression of Cre and/or rtTA-M2) for the production of the HC-Ad. A commercial plasmid in which the gene encoding for tTS is expressed under the control of the CMV viral promoter was obtained. Given that this promoter can undergo long-term silencing, it was substituted with the EF1a promoter, obtaining a more sustained expression in stable lines. Figure 4 shows the result of a RT-PCR to check the expression of the gene introduced after selecting the clones in 293 cells.

In addition, a sequence encoding for a fusion protein consisting of Cre recombinase bound at its carboxy and amino terminal end to respective mutated estrogen receptors, has been obtained. It has been described that this protein (called MerCreMer) only presents recombinase activity characteristic of Cre in the presence of tamoxifen (Verrou et al., 1999, Biol Chem. 380:1435-8). Therefore, in the event that the inducible tet-on system cannot obtain efficient control of the expression of Cre in the absence of doxycycline, the enzyme will be inactive and will allow the production of the helper virus. In contrast, when producing HC-Ad, tamoxifen will be added in the cells without tTS treated with doxycycline, such that the activation of the system will be maximum.

Once the construction of the plasmids integrating the regulation system for the Cre or MerCreMer recombinases was complete, the operation of the inducible system was verified. Figure 5 shows as an example the control of the expression of Cre in 293 cells. When the individual plasmids encoding for the transactivator and for Cre with the inducible promoter are co-transfected, expression of the recombinase in the presence of doxycycline is obtained, while both plasmids do not express separately. The plasmid containing the complete system also produces Cre, although to a lesser extent. Figure 5B shows how the latter plasmid only expresses considerable amounts of Cre when the cells are treated with doxycycline.

### EXAMPLE 3

### Helper adenoviral vectors expressing Cre in a manner regulated by doxycycline and tamoxifen

The process of constructing the self-inactivatable vector carrier of MerCreMer recombinase then began by means of incorporating the different elements in a plasmid containing the genome of a first generation adenoviral vector. The genomic structure of this vector is shown in Figure 6, along with the expected mechanism of operation. However, the complete system cannot be obtained in a single plasmid, because excision of the packaging sequence flanked by LoxP sites during the course of amplification of the plasmids in E. Coli was observed. To that end, the final step for obtaining the new vector consisted of the co-transfection in 293tTS cells of two plasmids which restore the whole genome after producing a homologous recombination in the cells (Figure 7). The virus was amplified in 293tTS cells and purified according using the same process as any other first generation adenoviral vector.

Once the virus was obtained, it was checked by means of Western Blot that it is capable of expressing the MerCreMer protein in a manner inducible by doxycycline (Figure 8). Its behavior in the presence of doxycycline and tamoxifen in different types of cells was then studied. To that end, 293, 293tTS or 293 cells constitutively expressing Cre recombinase (293Cre4) were infected (Parks et al., 1996, Proc. Natl. Acad. Sci. USA, 93:13565-70), in the presence or absence of the mentioned treatments. After 48 hours of infection the amount of infective virus produced in each case was quantified. As can be seen in Figure 9, viral product was drastically reduced in 293tTS cells only in the presence of doxycycline and tamoxifen, which is consistent with the expected inhibition of encapsidation of the virus due to excision of its encapsidation sequence. In the 293 cells, in which the level of basal expression of MerCreMer can be higher, the independent addition of doxycycline or tamoxifen caused a relative reduction of virus production, although again the combined treatment is what achieves a more notable reduction. When 293Cre4 cells are used, as is expected, viral production is low independently of the activation of the inducible system. To demonstrate the specificity of the system, a similar study was conducted, this time comparing the AdTetCre virus with a standard helper virus (AdLC8cluc), which has the encapsidation signal flanked by LoxP sites but does not express any recombinase (Parks et al., 1996, *supra.).* As can be seen in Figure 10, treatment with tamoxifen and doxycycline did not cause a significant change in the levels of production of the virus. The packaging of the virus is reduced only when 293Cre4 cells are used. In contrast, a drastic reduction is confirmed in the case of AdTetCre, with levels that are even lower than those observed with AdLC8cluc in 293Cre4 cells. To demonstrate that this reduction is due to the excision of the packaging sequence, the initial region of AdTetCre viruses present in 293 cells treated with doxycycline and tamoxifen was amplified by means of PCR. As can be seen in Figure 11A, the amplified fragment is smaller than that corresponding to the non-modified genome, and the size is compatible with the complete loss of the packaging sequence. This was further confirmed by means of sequencing. Figure 11B shows how the packaging sequence is lost in the case of a standard helper virus (AdLC8cluc) when it infects 293Cre4 cells. It can be further seen how this excision does not seem to be complete, contrary to the AdTetCre virus.

### EXAMPLE 4

### Production of gutless adenovirus by means of the use of the self-inactivatable helper adenovirus

Once it has been checked that the AdTetCre virus is inactivatable in an inducible manner (self-inactivatable), its capacity to function as a helper virus in the production process of an HC-Ad was then studied. To that end a standard protocol for the transfection of the plasmid encoding for HC-Ad (in this case KCZ, expressing the LacZ gene) and subsequent infection with the helper virus was followed. Then successive amplification steps are followed in which the HC-Ad produced in the previous step is used to infect a new plate of cells, together with the addition of new helper virus. The size and the number of plates progressively increase. This process was carried out both in 293 cells and in 293Cre4 cells with the AdTetCre virus as a helper. To compare with the standard method, the AdLC8cLuc helper virus was used in the same manner in 293Cre4 cells. The amount of ineffective units corresponding to the HC-Ad and the helper virus was quantified in each step. As can be seen in Figure 12A, the production of HC-Ad using the AdTetCre virus progressively increased in the successive steps carried out in 293Cre4 cells, while the contamination with helper adenovirus was always maintained well under that. In the last pass carried out in this example (pass 6), an amount of high-capacity adenovirus of 1.2x10¹¹ total IU, with a helper adenovirus contamination less than 0.05%. This yield and purity is considered acceptable for a mid-size production of HC-Ad. In contrast, when the AdLC8cluc virus was used, the degree of contamination with this virus increased throughout the steps, eventually exceeding the amount of high-capacity vector (Figure 12C). Although on other occasions this contamination is less and it is possible to use the standard method for the production of HC-Ad, the excessive accumulation of helper virus has frequently been described and indicates the lack of robustness of this method. With regard to the production in 293 cells with the AdTetCre virus, the degree of contamination was also too high (Figure 12B). This indicates that the Cre recombinase expressed by the packaging cells collaborates with the MerCreMer recombinase expressed by the virus to achieve an optimal effect. It is possible that this constitutive expression is important for assuring the presence of recombinase in initial moments after the infection with the helper virus. In several successive experiments carried out to date, the amount of AdTetCre virus has always remained under the amount of high-capacity virus when 293Cre4 cells have been used, which suggests greater reliability of this production method compared to the technology based on conventional helper adenoviruses.

### EXAMPLE 5

### Production of gutless adenovirus carrying a therapeutic transgene by means of using self-inactivating helper adenovirus

Once it has been checked that the AdTetCre virus is inactivatable in an inducible manner (self-inactivating) and has the capacity to function as a helper virus in the process for producing an HC-Ad, its capacity to produce an HC-Ad carrying a therapeutic gene was studied. For example, the GL-Ad/RUmIL-12 virus (also called HC-Ad-mIL-12) expresses the murine interleukin 12 (mIL-12) cytokine under the control of a Mifepristone-activatable liver-specific inducible promoter. This vector has shown antitumor effects in several animal liver cancer models (Wang et al., Gastroenterology 2004, 126: 278-289). The pGLAd-RUmIL-12 plasmid containing the genome of the GL-Ad/RUmIL-12 was produced as has been previously described (Wang, L., et al., 2004 Gastroenterology 126: 278-89), it was digested with the PmeI restriction enzyme in order to linearize it and eliminate the resistance to the antibiotic. It was subsequently precipitated by adding sodium acetate (300 mM) and 2.5 volumes of absolute ethanol. The sample was kept at -20°C for at least 1 hour, and then centrifuged at 11,000 g for 30 minutes at 4°C, the precipitate was washed with 70% ethanol and air-dried. It was resuspended in water and 15 µg were used to transfect 1x10⁶ 293Cre4 cells in a 60 mm plate which had been previously treated for 24 hours with tamoxifen and doxycycline. The transfection was carried out using 20 µl of Lipofectamine 2000 (Invitrogen). The medium was changed after 6 hours, adding DMEN medium with 2% FBS and the AdTetCre virus at an MOI of 1 virus/cell. This MOI was determined in a prior experiment in which the amount of virus necessary to produce 90% of the cytopathic effect after 48 hours of infection was assayed. The infection was left to progress for 48 hours and the cells were then collected in 200 µl of 10 mM Tris, pH 8.1. This transfection-infection step is called passage 0. The virus produced in this phase was released from the cells by means of 3 freezing/thawing cycles and 150 µl of the lysate were used to infect a new plate of 293Cre4 cells along with an MOI 1 of the AdTetCre helper virus. As in the previous case, the cells were pretreated for 24 hours with Doxycycline and tamoxifen. The passages referred to as passages 1-3 are carried out in this way. In passage 4, two 150 mm plates are infected with 1 x 10⁷ 293Cre4 cells in each one, in passage 5 there are twenty 150 mm plates, and the final passage of the medium-scale production consists of thirty 150 mm plates with the lysate equivalent to four 150 mm plates. The produced virus was purified by means of a double cesium chloride gradient and the salt was eliminated from the preparation by means of a size exclusion column (Sephadex). The amount of AdTetCre was quantified in each of the passages by means of the commercial AdenoX rapid titer kit (BD Biosciences), according to the manufacturer's specifications. Production of GL-Ad/RumIL-12 reached the capacity of 1.5x10¹² viral particles (vp), with an AdTetCre helper adenovirus contamination less than 2.6x10⁶ iu.

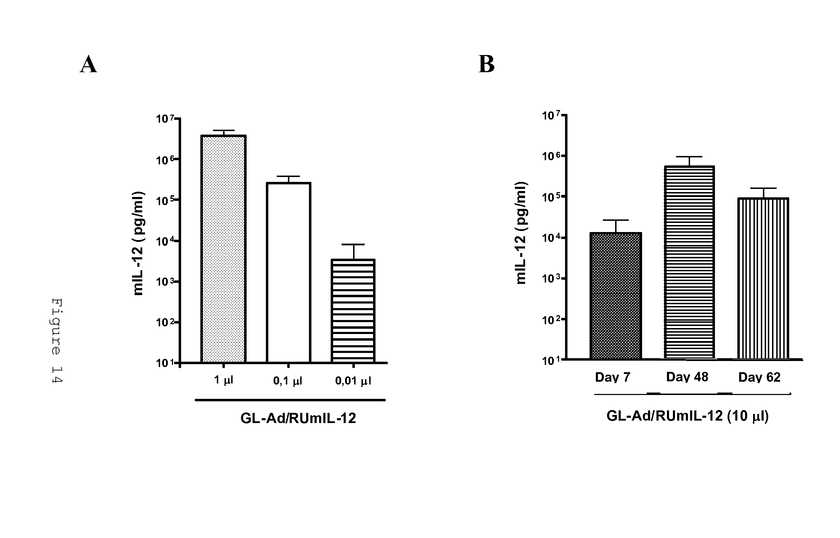

Given that this vector does not express any reporter gene, its function was verified by means of determining the production of the transgene (mIL-12) in infected cells. Figure 14A shows the mIL-12 levels obtained after infecting HuH-7 cells (derived from a human hepatocarcinoma) with different amounts of the high-capacity adenoviral vector GL-Ad/RUmIL-12 in the presence of the Mifepristone inducer. Subsequently, the function of the vector was verified *in vivo.* To that end, the vector was endogenously injected in mice, and the amount of mIL-12 present in the serum after administering the Mifepristone inducer intraperitoneally was measured. The induction and measurement of mIL-12 were carried out 7, 48 and 62 days after the injection of the vector, and it was checked that the mice stably express high amounts of the transgene (Figure 14B). This data shows that the novel method is efficient for the production of vectors of clinical interest.

## Claims

1. A polynucleotide comprising:
a) a region encoding a site-specific recombinase, wherein said region is under operative control of an inducible promoter and
b) an essential region for the replication and/or for the packaging of the adenoviral genome flanked by recognition sequences specific for the recombinase encoded by sequence a) wherein said recognition sequences are oriented such that the excision of said essential region occurs in the presence of said recombinase.

2. A polynucleotide according to claim 1, wherein the essential region (b) for the packaging or the replication of the adenoviral genome is the packaging signal ψ.

3. A polynucleotide according to claims 1 or 2, additionally comprising a sequence encoding a transcriptional activator which is capable of specifically activating the promoter regulating the expression of the recombinase encoded by sequence (a) in the presence of a ligand of said transcriptional activator.

4. A polynucleotide according to claim 3, wherein said transcriptional activator is a tetracycline analog-dependent transactivator.

5. A polynucleotide according to claim 4, wherein the transcriptional activator is the rtTA-M2 transactivator.

6. A polynucleotide according to any of the preceding claims, wherein the recombinase is Cre or a fusion protein comprising the sequence of the Cre recombinase.

7. A polynucleotide according to claim 6, wherein the recombinase additionally comprises a hormone binding domain or an activatable nuclear localization sequence, preferably the hormone binding domain of the estrogen receptor.

8. An expression vector comprising a polynucleotide according to any of claims 1 to 7.

9. An expression vector according to claim 8, wherein said vector is an adenoviral vector.

10. An adenoviral particle comprising the sequence of a polynucleotide according to any claims 1 to 7.

11. A system for the replication and packaging of a self-inactivating helper adenovirus, comprising:
a) a polynucleotide according to any of claims 1 to 7, or an expression vector according to claims 8 or 9, or an adenoviral particle according to claim 10; and
b) a eukaryotic cell.

12. A system according to claim 11, wherein the cell forming component (b) comprises a promoter-specific transcriptional repressor regulating the expression of the site-specific recombinase.

13. A system according to claim 12, wherein the transcriptional repressor is active in the absence of a ligand which is capable of promoting the expression of the site-specific recombinase.

14. A method for obtaining a self-inactivating helper adenovirus comprising
(i) contacting a cell with a polynucleotide according to any of claims 1 to 7, an expression vector according to claims 8 or 9, or an adenoviral particle according to claim 10 and, optionally, with one or several polynucleotides encoding the adenoviral proteins necessary for the packaging and replication of the adenovirus in the cell in suitable conditions so that the entry in the cell of the adenoviral genome or of the polynucleotide comprising said genome occurs,
(ii) keeping the cell in suitable conditions to allow the packaging and replication of the adenovirus in the cell and to prevent the expression of the site-specific recombinase and
(iii) recovering the adenoviruses from the medium.

15. A method according to claim 14, wherein the cell comprises a promoter-specific transcriptional repressor regulating the expression of the site-specific recombinase wherein said repressor is active in the absence of an analog of a ligand capable of activating the promoter regulating the expression of the site-specific recombinase.

16. A method according to claim 15, wherein said transcriptional repressor is the doxycycline-dependent tTS repressor.

17. A helper adenovirus obtained according to the method of claim 16.

18. Use of a polynucleotide according to any of claims 1 to 7 or of a vector according to claims 8 or 9 or of an adenoviral particle according to claim 10 comprising said polynucleotide for the production and packaging of high-capacity adenovirus.

19. A system for the production of a high-capacity recombinant adenovirus comprising
(a) a polynucleotide according to any of claims 1 to 7, or an expression vector according to claim 8 or 9, or an adenoviral particle according to claim 10;
(b) an adenovirus the genome of which comprises a gene of interest and which lacks the sequence encoding at least one of the essential proteins for the replication or at least one of the essential proteins for the packaging of said adenovirus or with a polynucleotide comprising the genome of said adenovirus and
(c) a eukaryotic cell.

20. A system according to claim 19, wherein the eukaryotic cell comprises a polynucleotide expressing the site-specific recombinase which is encoded by component (a) of the system.

21. A system according to claim 20, wherein the region encoding a site-specific recombinase is under operative control of an inducible promoter.

22. A method for generating high-capacity adenovirus expressing a gene of interest comprising the steps of
(i) contacting a cell with an adenovirus in suitable conditions so that the entry in the cell of the genome of said adenovirus or of the polynucleotide comprising said genome occurs,
(ii) contacting said cell with a second component selected from the group of:
(a) a polynucleotide comprising:
1. a region encoding a site-specific recombinase, wherein said region is under operative control of an inducible promoter and
2. an adenoviral packaging signal ψ flanked by recognition sequences specific for the recombinase encoded by sequence a) wherein said recognition sequences are oriented such that the excision of the packaging signal ψ occurs in the presence of said recombinase,
(b) an adenoviral vector comprising a polynucleotide according to (a) and
(c) an adenoviral particle comprising a polynucleotide according to (a)
in suitable conditions so that the entry in the cell of said polynucleotide, of said adenoviral vector or of the genome of said adenoviral particle occurs,
(iii) keeping the cell in suitable conditions for the expression of the proteins encoded by the genomes of both viruses, for the replication of the genomes of both adenoviruses and for the expression of the recombinase encoded by the polynucleotide, vector or adenovirus used in step (ii) and
(iv) recovering the high-capacity adenovirus from the cell culture.

23. A method according to claim 22, wherein the polynucleotide defined in (a) and/or the cell additionally comprises a sequence encoding a specific transactivator of the promoter regulating the expression of the site-specific recombinase.

24. A method according to claim 23, wherein steps (i) and/or (ii) are carried out in the presence of a ligand which is capable of binding and activating the specific transactivator of the promoter regulating the expression of the site-specific recombinase.

25. A method according to claim 24, wherein said transactivator is rtTA-M2 and wherein said ligand is doxycycline.

26. A method according to any of claims 22 to 25, wherein the cell comprises a sequence encoding a site-specific recombinase wherein said recombinase is the same which is encoded by the polynucleotide defined in (a).

27. A method according to any of claims 22 to 26, wherein the site-specific recombinase is Cre or a fusion protein comprising the sequence of the Cre recombinase.

28. A method according to claim 27, wherein the recombinase is merCremer.

29. A method according to claim 28, wherein step (i) and/or (ii) is carried out in the presence of tamoxifen.

30. A method according to any of claims 22 to 29, comprising repeating at least once steps (i) to (iv) using in step (i) of each cycle the adenovirus recovered in step (iv) of the previous cycle, and optionally adding a new dose of helper adenovirus in steps (i) of each cycle.
